# EUROPEAN PATENT APPLICATION

(11) **EP 4 721 758 A1**
(43) Date of publication of application: **08.04.2026**
(21) Application number: 24815526.9
(22) Date of filing: 29.05.2024
(51) Int. Cl.: A61K 45/00, A61K 31/404, A61K 31/4155, A61K 31/428, A61K 31/4704, A61K 31/4745, A61K 31/4985, A61K 31/506, A61K 31/519, A61K 31/5375, A61K 31/69, A61K 31/713, A61K 45/06, A61K 48/00, A61P 35/00, C12Q 1/6841, C12Q 1/686, G01N 33/53

(54) **NOVEL MEDICINE FOR TREATING OSTEOSARCOMA**

(30) Priority: 30.05.2023 JP 2023088672
(71) Applicant: Japanese Foundation For Cancer Research, Tokyo 135-8550 (JP)
(72) Inventor: TAKAGI, Satoshi, Tokyo 135-8550 (JP); KATAYAMA, Ryohei, Tokyo 135-8550 (JP)
(74) Representative: Plasseraud IP
(86) International application number: PCT/JP2024/019715
(87) International publication number: WO 2024/248045

(57) **Abstract**

The present invention aims to develop a new pharmaceutical composition for treating osteosarcoma. Another object of the present invention is to develop a therapeutic marker for examiniing the therapeutic effect of a pharmaceutical composition when treating osteosarcoma.

In order to solve the above-mentioned problems, the present inventors have found compounds that can suppress osteosarcoma cell proliferation, and, therefore, have demonstrated that a new pharmaceutical composition for treating osteosarcoma can be provided. In addition, the present inventors have also found that osteosarcoma, which is the target of treatment with the new pharmaceutical composition, is a cell in which a copy number of the MCL1 gene is amplified or in which a production of the Mcl-1 protein is increased, or a cell in which a copy number of the BCL2L1 gene is amplified or in which a production of the Bcl-xL protein is increased, and, therefore, have demonstrated that, detection of these can be used as therapeutic markers for examining the therapeutic effect of the new pharmaceutical composition when treating osteosarcoma.

## Description

### Technical Field

The present invention relates to a novel pharmaceutical composition for treating osteosarcoma. The present invention also relates to the development of a therapeutic marker for examining the therapeutic effect of a pharmaceutical composition for treating osteosarcoma.

### Background Art

Osteosarcoma is the most common primary malignant bone tumor, occurring mainly in infants, adolescents, and young adults. Treatment of osteosarcoma in combination with surgery and chemotherapy, consisting of a four-drug combination of adriamycin (DOX), cisplatin (CDDP), high-dose methotrexate (MTX), and ifosfamide, was established in the 1970s and remains the standard treatment today (Non-Patent Document 1, Non-Patent Document 2).

With the establishment of this multidisciplinary treatment, the 5-year overall survival rate of patients without distant metastasis at the time of initial diagnosis has reached approximately 70%. However, there are few effective treatments for patients with metastatic or recurrent osteosarcoma, and the 5-year survival rate is only 20% to 30%, and the prognosis is poor. Furthermore, the established treatment consisting of multidrug combination therapy and surgical resection has significant toxicity and morbidity. A phase III clinical trial of intensive postoperative chemotherapy in high-risk groups did not improve event-free survival period but increased toxicity (Non-Patent Document 3).

Furthermore, recent clinical trials have shown that immune checkpoint inhibitors, including pembrolizumab, nivolumab, and/or ipilimumab, have no effectivity against osteosarcoma (Non-Patent Document 4, Non-Patent Document 5), highlighting the continued critical need for new therapeutic strategies.

### Citation List

### Non Patent Literature

Non Patent Literature 1: Link, M. P. et al. N Engl J Med 314, 1600-1606, (1986)
Non Patent Literature 2: Meyers, P. A. et al. J Clin Oncol 10, 5-15, (1992).
Non Patent Literature 3: Marina, N. M. et al. Lancet Oncol 17, 1396-1408, (2016).
Non Patent Literature 4: D'Angelo, S. P. et al. Lancet Oncol 19, 416-426, (2018).
Non Patent Literature 5: Tawbi, H. A. et al. Lancet Oncol 18, 1493-1501, (2017).

### Summary of Invention

### Technical Problem

The present invention aims to develop a novel pharmaceutical composition for treating osteosarcoma. Another object of the present invention is to develop a therapeutic marker for examining the therapeutic effect of a pharmaceutical composition when treating osteosarcoma.

### Solution to Problem

In order to solve the above-mentioned problems, the present inventors have found compounds that can suppress osteosarcoma cell proliferation, and, therefore, have demonstrated that a new pharmaceutical composition for treating osteosarcoma can be provided. In addition, the present inventors have also found that osteosarcoma, which is the target of treatment with the new pharmaceutical composition, is a cell in which a copy number of the MCL1 gene is amplified or in which a production of the Mcl-1 protein is increased, or a cell in which a copy number of the BCL2L1 gene is amplified or in which a production of the Bcl-xL protein is increased, and, therefore, have demonstrated that, detection of these can be used as therapeutic markers for examining the therapeutic effect of the new pharmaceutical composition when treating osteosarcoma.

More specifically, the present application provides the following aspects to solve the above-mentioned problems:
[1]: A pharmaceutical composition for treating osteosarcoma, comprising an Mcl-1 inhibitor.
[2]: The pharmaceutical composition according to [1], wherein the Mcl-1 inhibitor is selected from the group consisting of obatoclax, AZD5991, MIK665, siRNA against the MCL1 gene, or a combination thereof.
[3]: The pharmaceutical composition according to [1] or [2], wherein the osteosarcoma is one in which a copy number of the MCL1 gene is amplified or one in which a production of the Mcl-1 protein is increased.
[4]: The pharmaceutical composition according to [3], wherein the copy number of the MCL1 gene in the cells is measured by PCR or FISH.
[5]: The pharmaceutical composition according to [3], wherein the production of Mcl-1 protein in the cells is measured by immunoblotting or immunohistochemical staining.
[6]: A pharmaceutical composition for treating osteosarcoma in which an Mcl-1 inhibitor is ineffective, comprising a Bcl-xL inhibitor.
[7]: The pharmaceutical composition according to [6], wherein the Bcl-xL inhibitor is selected from the group consisting of A-1331852, an siRNA against the BCL2L1 gene, or a combination thereof.
[8]: The pharmaceutical composition according to [6] or [7], wherein the Mcl-1 inhibitor is selected from the group consisting of obatoclax, AZD5991, MIK665, siRNA against the MCL1 gene, or a combination thereof.
[9]: The pharmaceutical composition according to [6] or [7], wherein the osteosarcoma is a cell in which a copy number of the BCL2L1 gene is amplified or one in which a production of the Bcl-xL protein is increased.
[10]: The pharmaceutical composition according to [9], wherein the copy number of the BCL2L1 gene in the cells is measured by PCR or FISH.
[11]: The pharmaceutical composition according to [9], wherein the production of Bcl-xL protein in the cells is measured by immunoblotting or immunohistochemical staining.
[12]: A pharmaceutical composition for treating osteosarcoma, comprising a component selected from the group consisting of obatoclax, tipifarnib, bortezomib, carfilzomib, SN-38, A-1331852, and derivatives thereof, or combinations thereof.
[13]: The pharmaceutical composition according to [12], wherein the osteosarcoma is a cell in which a copy number of the MCL1 gene is amplified or in which a production of the Mcl-1 protein is increased, or a cell in which a copy number of the BCL2L1 gene is amplified or in which a production of the Bcl-xL protein is increased.
[14]: The pharmaceutical composition according to [13], wherein the copy number of the MCL1 gene or the copy number of the BCL2L1 gene in the cells is measured by PCR or FISH.
[15]: The pharmaceutical composition of [13], wherein the production of Mcl-1 protein or Bcl-xL protein in the cells is measured by immunoblotting or immunohistochemical staining.
[16]: A pharmaceutical composition for treating osteosarcoma, comprising an Mcl-1 inhibitor in combination with a Bcl-xL inhibitor or an IGF-1R inhibitor.
[17]: The pharmaceutical composition of [16], wherein the Mcl-1 inhibitor is selected from the group consisting of obatoclax, AZD5991, MIK665, siRNA against the MCL1 gene, or a combination thereof.
[18]: The pharmaceutical composition according to [16], wherein the Bcl-xL inhibitor is selected from the group consisting of A-1331852 and siRNA against the BCL2L1 gene.
[19]: The pharmaceutical composition according to [16], wherein the IGF-1R inhibitor is selected from the group consisting of OSI906, AEW541, AZD3463 and siRNA against the IGF-1R gene.
[20]: The pharmaceutical composition according to any one of [16] to [19], wherein the osteosarcoma is a cell in which a copy number of the MCL1 gene is amplified or in which a production of the Mcl-1 protein is increased, or a cell in which a copy number of the BCL2L1 gene is amplified or in which a production of the Bcl-xL protein is increased.
[21]: The pharmaceutical composition according to [20], wherein the copy number of the MCL1 gene or the BCL2L1 gene in the cells is measured by PCR or FISH.
[22]: The pharmaceutical composition according to [20], wherein the production of the Mcl-1 protein or the Bcl-xL protein in the cells is measured by immunoblotting or immunohistochemical staining.
[23]: A method for detecting an increase in the copy number of the MCL1 gene or an increase in the production of the Mcl-1 protein, an increase in the copy number of the BCL2L1 gene, or an increase in the production of the Bcl-xL protein in osteosarcoma tissue or blood collected from a patient suffering from osteosarcoma, which is an indicator of whether or not the patient is a target for administration of a pharmaceutical composition comprising a component selected from the group consisting of an Mcl-1 inhibitor, a Bcl-xL inhibitor, obatoclax, tipifarnib, bortezomib, carfilzomib, SN-38, A-1331852, and derivatives thereof, or a combination thereof, or comprising an Mcl-1 inhibitor in combination with a Bcl-xL inhibitor or an IGF-1R inhibitor.
[24]: The method according to [23], wherein the copy number of the MCL1 gene or the BCL2L1 gene in the cells is measured by PCR or FISH.
[25]: The method according to [23], wherein the production of the Mcl-1 protein or the Bcl-xL protein in the cells is measured by immunoblotting or immunohistochemical staining.

### Advantageous Effects of Invention

The present invention has demonstrated that compounds capable of suppressing osteosarcoma cell proliferation can be found, and, therefore, can provide a new pharmaceutical composition for treating osteosarcoma. In addition, it has been found that osteosarcoma, which is the target of treatment with the new pharmaceutical composition, is a cell in which a copy number of the MCL1 gene is amplified or in which a production of Mcl-1 protein is increased, or a cell in which a copy number of the BCL2L1 gene is amplified or in which a production of Bcl-xL protein is increased, and it has also been demonstrated that detection of these can be used as therapeutic markers for examining the therapeutic effect of the new pharmaceutical composition when treating osteosarcoma.

### Brief Description of Drawings

[Fig. 1A] Fig. 1A shows the drug sensitivity profiles of osteosarcoma cell lines and PDC using an inhibitor library.
[Fig. 1B] Fig. 1B shows a bar graph of the cell viability values of each cell line after treatment with obatoclax or navitoclax.
[Fig. 1C] Fig. 1C shows the protein expression levels of Bcl-2 family proteins in osteosarcoma cells.
[Fig. 1D] Fig. 1D shows changes in the copy number of MCL1 gene and BCL2L1 gene in osteosarcoma cells.
[Fig. 1E] Fig. 1E shows the amplification of MCL1 in osteosarcoma cells.
[Fig. 2] Fig. 2 shows the effect of MIK665, an Mcl-1 inhibitor, on osteosarcoma cells.
[Fig. 3-1] Fig. 3-1 shows the effects of RNAi-mediated genetic knockdown in NOS-10 cells, Sa-xeno147 cells, and G-292 cells, confirmed by immunoblotting for various proteins.
[Fig. 3-2] Fig. 3-2 shows the efficiencies of RNAi-mediated genetic knockdown in Sa-xeno147 cells and G-292 cells, confirmed by qPCR for Bcl-2.
[Fig. 3-3] Fig. 3-3 shows the effects of siRNA on caspase-3/7 activity in osteosarcoma cells.

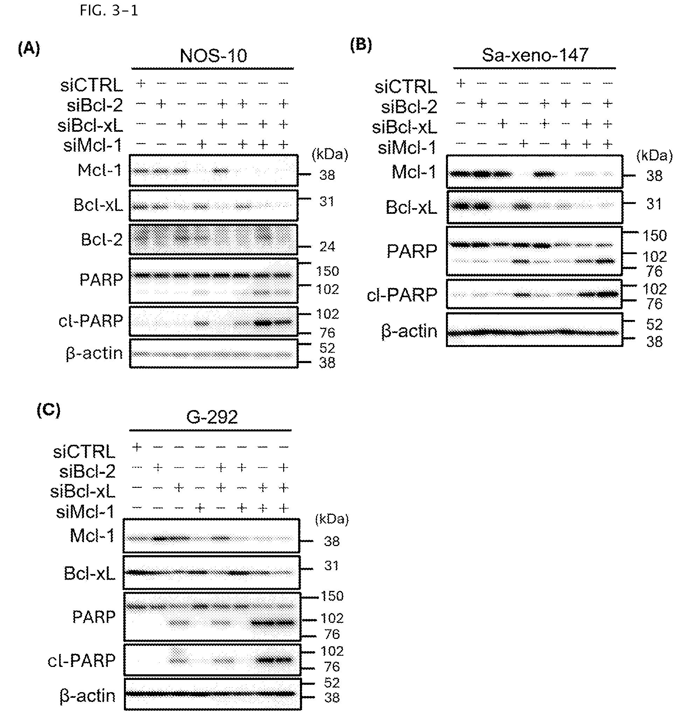

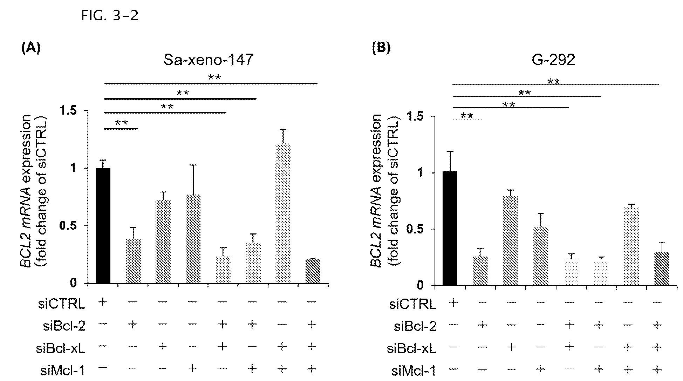

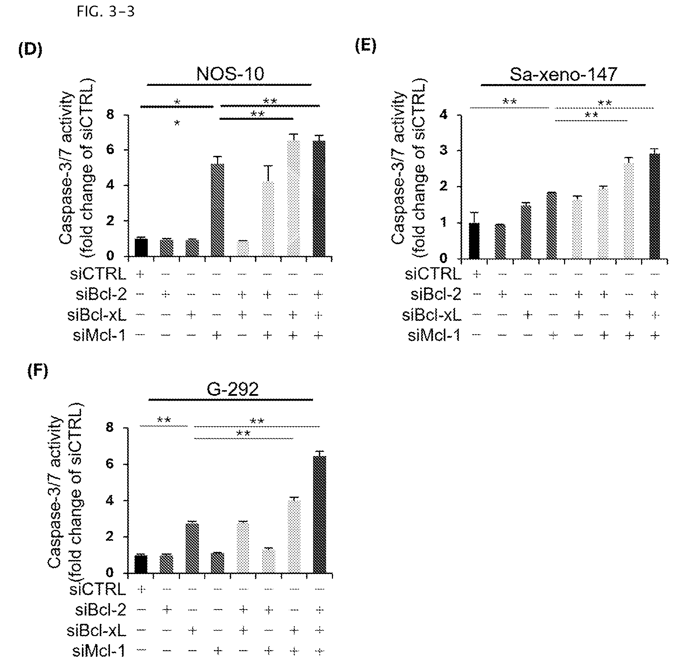

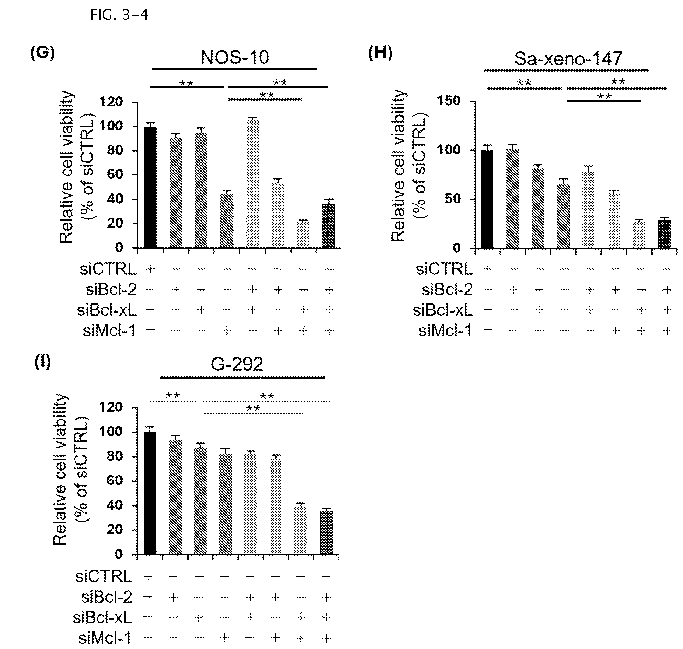

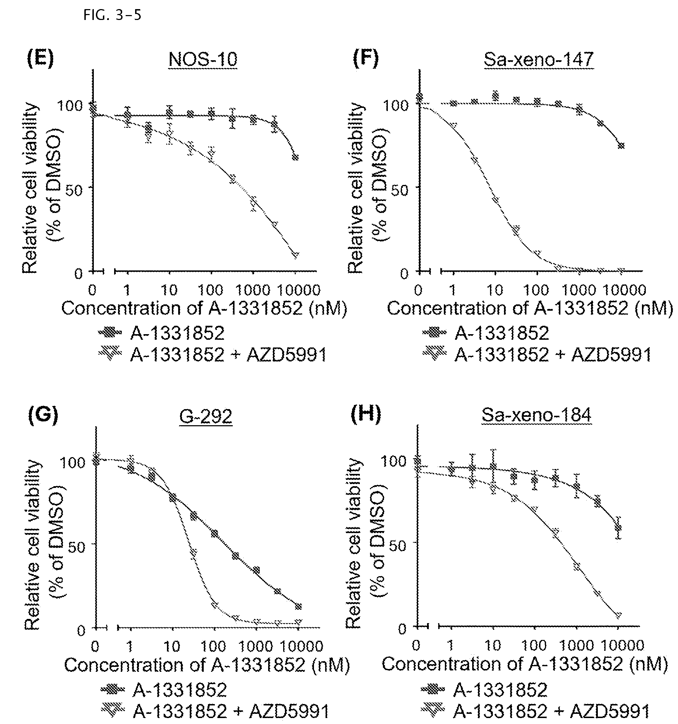

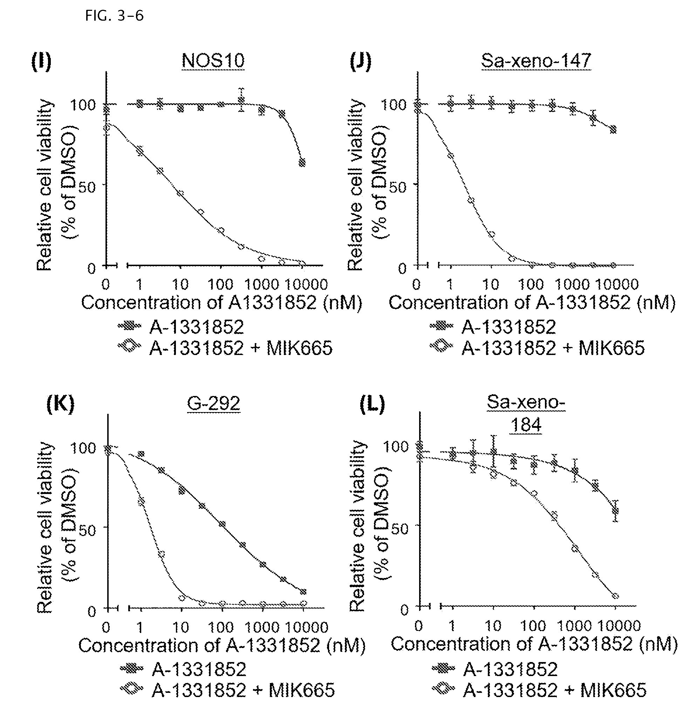

[Fig. 3-4] Fig. 3-4 shows the effects of siRNA on osteosarcoma cell viability.
[Fig. 3-5] Fig. 3-5 shows the concentration dependencies of cell viability of osteosarcoma cells when AZD5991 is used as an Mcl-1 inhibitor and A-1331852 is used as a Bcl-xL inhibitor.
[Fig. 3-6] Fig. 3-6 shows the concentration dependencies of cell viability of osteosarcoma cells when MIK665 is used as an Mcl-1 inhibitor and A-1331852 is used as a Bcl-xL inhibitor.

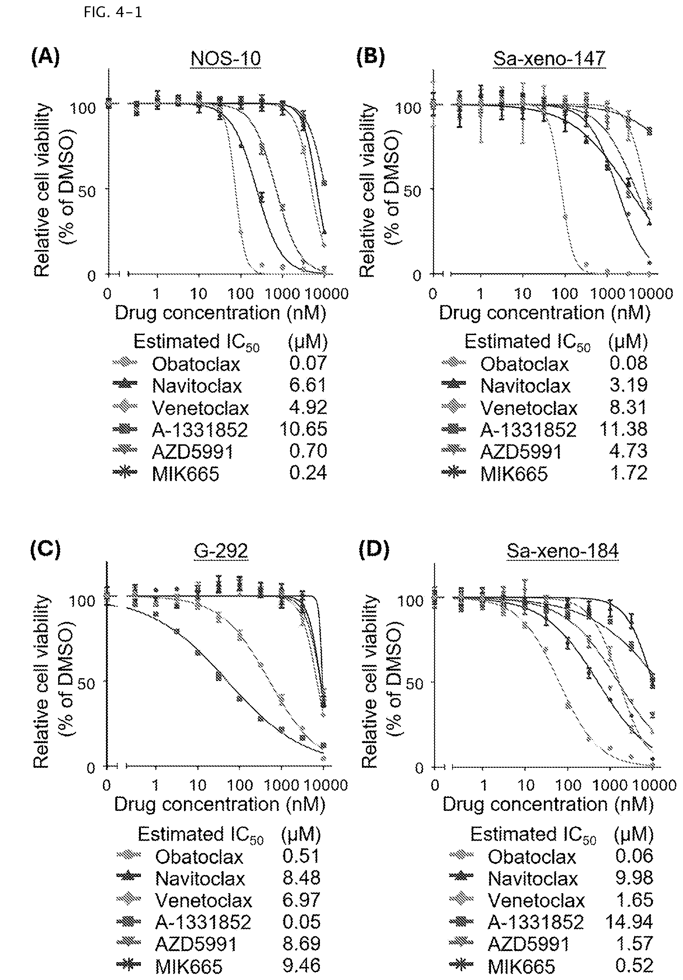

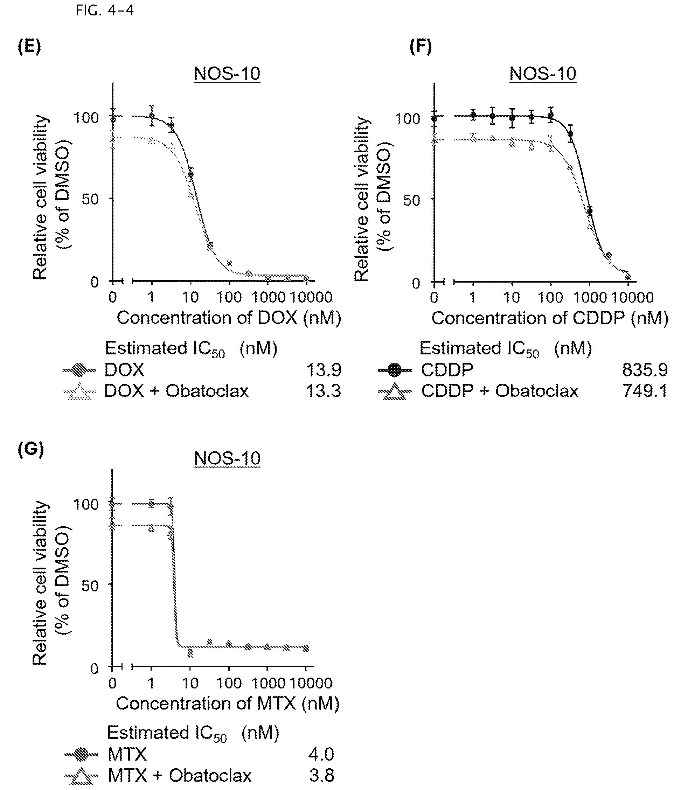

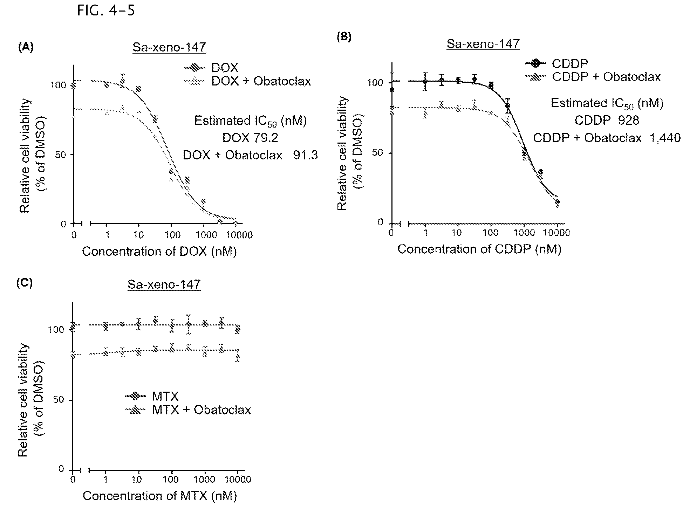

[Fig. 4-1] Fig. 4-1 shows the results of evaluating the drug sensitivities of obatoclax, navitoclax, venetoclax, A-1331852, AZD5991, or MIK665 in NOS-10 cells, Sa-xeno-147 cells, G-292 cells, and Sa-xeno-184 cells.

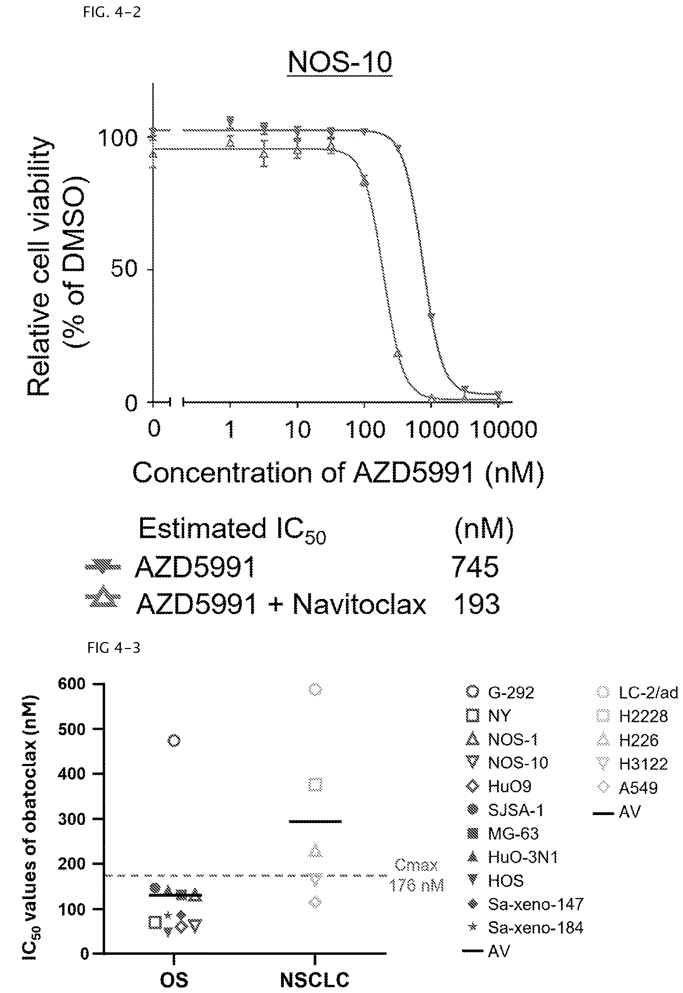

[Fig. 4-2] Fig. 4-2 shows the combined effects of AZD5991 and 3 µ(micro)M navitoclax on cell viability in NOS-10 cells.
[Fig. 4-3] Fig. 4-3 shows the IC50 value of obatoclax in osteosarcoma cell lines and non-small cell lung cancer cell lines.
[Fig. 4-4] Fig. 4-4 shows that the combined use of obatoclax with DOX, CDDP, or MTX does not induce a synergistic cytotoxic effect in NOS-10 cells.
[Fig. 4-5] Fig. 4-5 shows that the combined use of obatoclax with DOX, CDDP, or MTX does not induce a synergistic cytotoxic effect in Sa-xeno-147 cells.

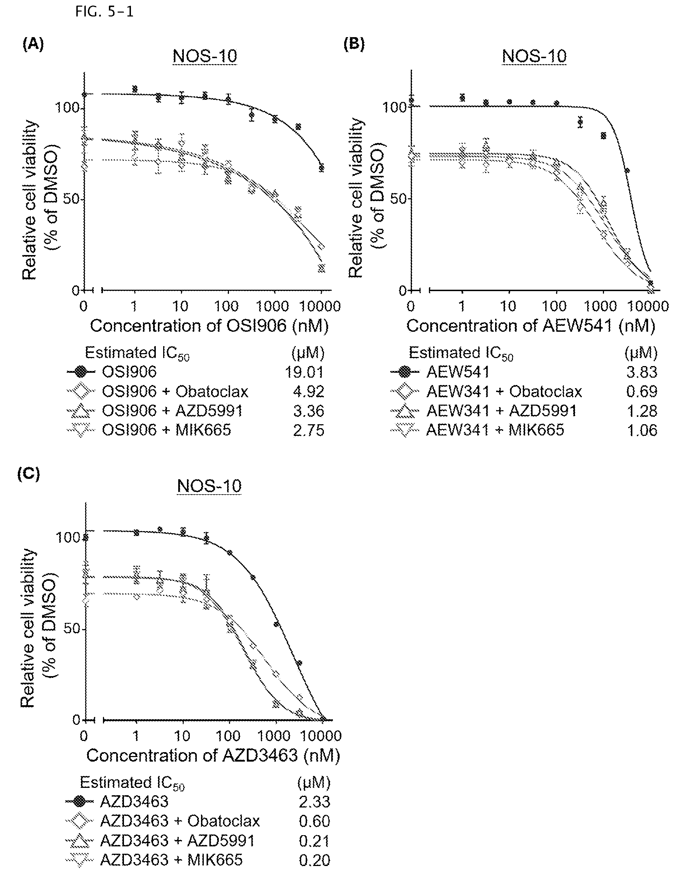

[Fig. 5-1] Fig. 5-1 shows that IGF-1R inhibitors such as OSI906, AEW541, and AZD3463 enhance the growth inhibitory effect when combined with Mcl-1 inhibitors such as obatoclax, AZD5991, or MIK665.

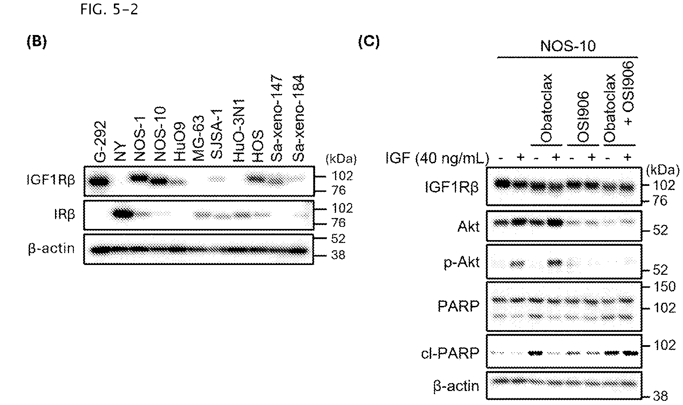

[Fig. 5-2] Fig. 5-2 shows the expression levels of IGF-1Rβ(beta) protein or insulin receptor (IR) β(beta) protein in osteosarcoma cell lines. The expression of each protein was detected using immunoblot using the indicated antibodies.

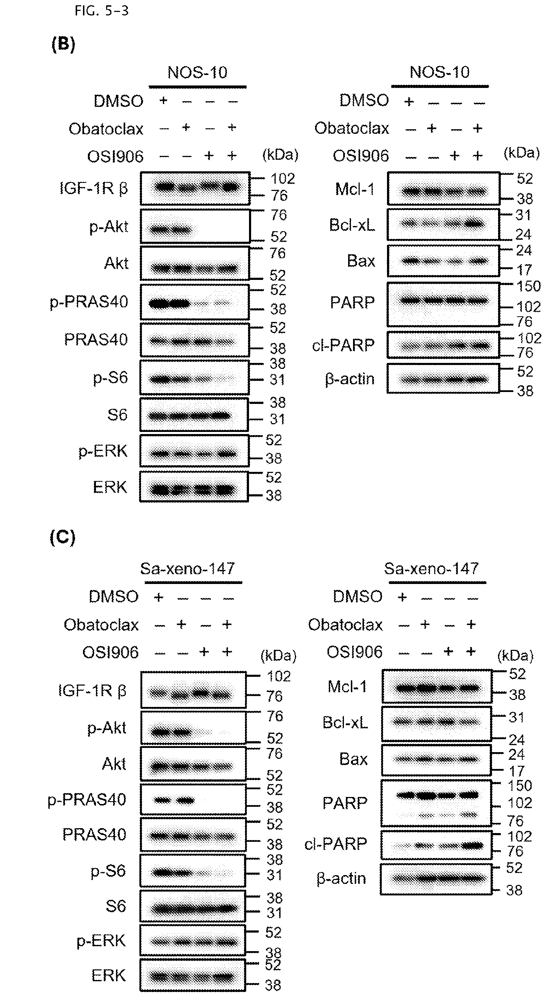

[Fig. 5-3] Fig. 5-3 shows the effect of the combined use of obatoclax and OSI906 on the expression of IGF1R downstream pathway and apoptosis-related proteins in NOS-10 cells and Sa-xeno-147 cells.

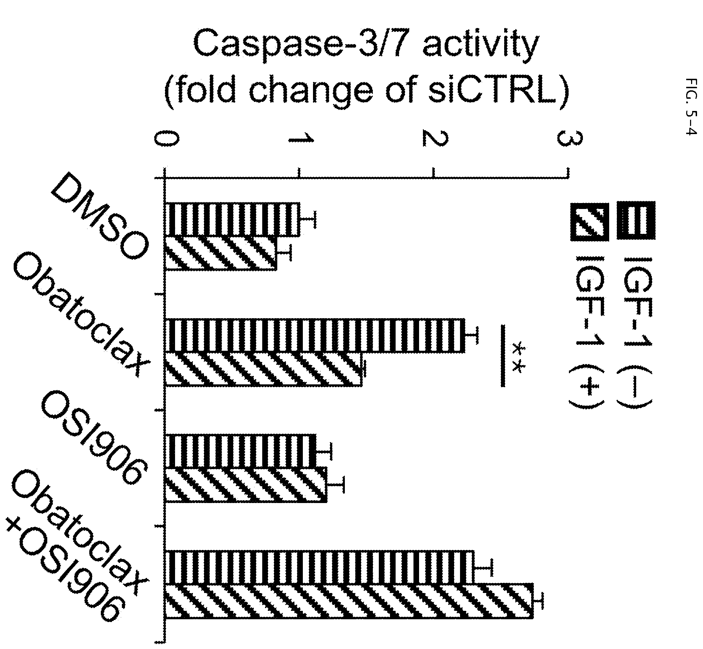

[Fig. 5-4] Fig. 5-4 shows the results in which activation of IGF signaling by the addition of IGF-1 significantly inhibits obatoclax-induced apoptosis and confers drug resistance to NOS-10 cells.

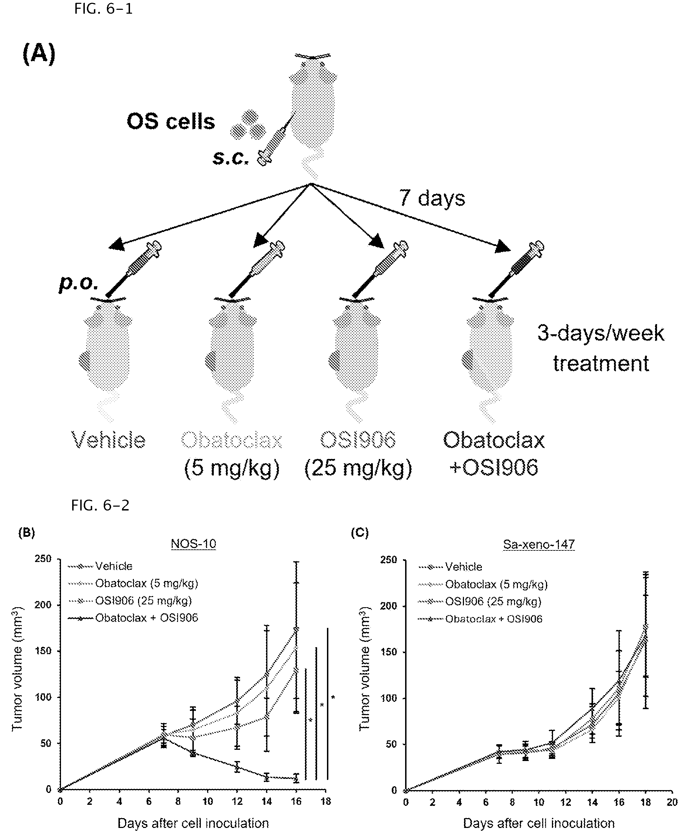

[Fig. 6-1] Fig. 6-1 shows the experimental scheme of this example when obatoclax is used as the Mcl-1 inhibitor.
[Fig. 6-2] Fig. 6-2 shows the changes in tumor volume in NOS-10 cells (B) and Sa-xeno-147 cells (C) after drug treatment when obatoclax is used as the Mcl-1 inhibitor.

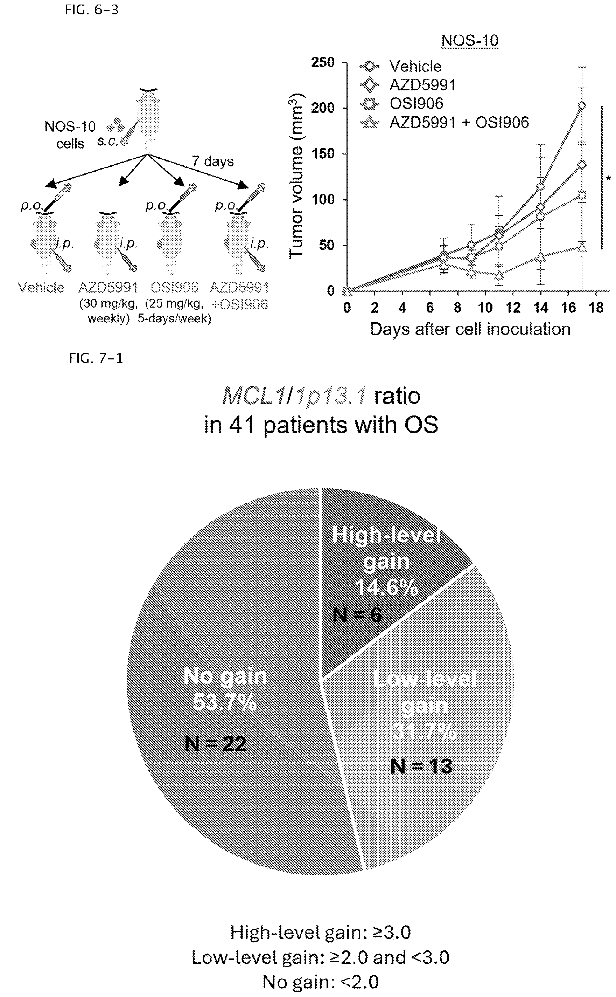

[Fig. 6-3] Fig. 6-3 shows the experimental scheme of this example when AZD5991 is used as the Mcl-1 inhibitor and the changes in tumor volume in NOS-10 cells due to the drug treatment.
[Fig. 7-1] Fig. 7-1 shows the classification of the MCL1 to 1p13.1 ratios in 41 osteosarcoma patients, where an MCL1/1p13.1 ratio of ≥ 3.0 is defined as high-level amplification group, and 2.0 ≤ an MCL1/1p13.1 ratio < 3.0 is defined as low-level amplification group.

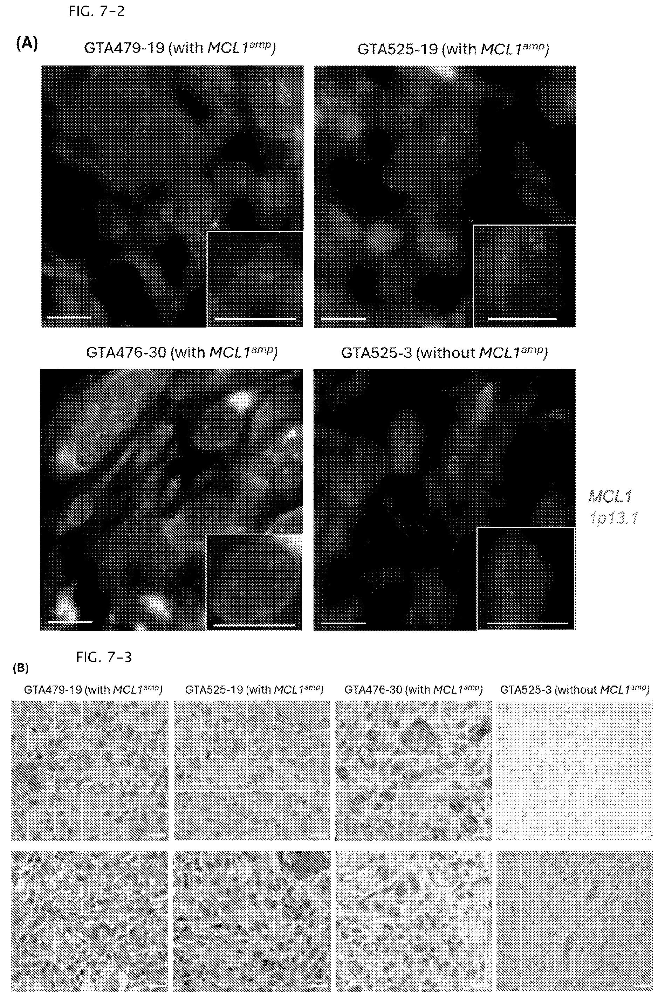

[Fig. 7-2] Fig. 7-2 shows the results of an examination of the copy number of MCL1 gene in osteosarcoma patients by FISH.
[Fig. 7-3] Fig. 7-3 shows the results of an examination of the MCL1 protein expression in osteosarcoma patients by immunohistochemical staining.

### Description of Embodiments

### Pharmaceutical composition for treating osteosarcoma with a single compound

In the present invention, a library of known compounds was screened for 11 types of osteosarcoma cell lines, and compounds capable of inhibiting cell proliferation of almost all osteosarcoma cells were selected. As a result, six molecular targeted drugs, i,e,m obatoclax, a BH3 mimetic; tipifarnib, a farnesyltransferase inhibitor; bortezomib and carfilzomib, proteasome inhibitors; SN-38, a topoisomerase inhibitor; and A-1331852, a Bcl-xL inhibitor, or Mcl-1 inhibitors, were identified as compounds capable of inhibiting cell proliferation of osteosarcoma cells.

Accordingly, in a first embodiment, the present invention provides a pharmaceutical composition for treating osteosarcoma, comprising an Mcl-1 inhibitor.

In a second embodiment, the present invention provides a pharmaceutical composition for treating osteosarcoma in which an Mcl-1 inhibitor is ineffective, comprising a Bcl-xL inhibitor.

In a third embodiment, the present invention provides a pharmaceutical composition for treating osteosarcoma, comprising a component selected from the group consisting of obatoclax, tipifarnib, bortezomib, carfilzomib, SN-38, A-1331852, and derivatives thereof, or combinations thereof.

The term Mcl-1 inhibitor means a substance capable of inhibiting the expression of the MCL1 gene or of inhibiting the production or function of the Mcl-1 protein. In the examples of the present application, obatoclax was obtained from a compound library as a result of screening the compound library using the reduction in cell viability of osteosarcoma cells, and it was revealed that the expression of the MCL1 gene is widely increased in osteosarcoma cells. Since obatoclax is an Mcl-1 inhibitor, it was confirmed whether Mcl-1 inhibitors other than obatoclax can also reduce the cell viability of osteosarcoma cells, and the two types of Mcl-1 inhibitors examined (AZD5991, MIK665) also showed a similar effect of reducing the cell viability of osteosarcoma cells.

In the pharmaceutical composition of the first embodiment of the present invention, the Mcl-1 inhibitor may be any of the known Mcl-1 inhibitors, such as obatoclax, AZD5991, MIK665, marinopyrrole A (maritoclax), VU661013, S63845, A-1210477, UMI-77, motixafortide (BL-8040), (R)-(-)-gossypol acetic acid, sabutoclax, TW-37, gambogic acid, and siRNA against the MCL1 gene.

Obatoclax is a compound known as a pan-Bcl-2 inhibitor and BH3 mimetic, and is a compound known to induce apoptosis in cancer cells such as hepatocellular carcinoma as an anticancer drug or BCL-2 inhibitor.

AZD5991 is a molecule known to be a highly active macrocyclic Mcl-1 inhibitor with affinity for Mcl-1 at subnanomolar concentrations.

MIK665 is an inhibitor of Mcl-1, a molecule known to have pro-apoptotic and antitumor properties.

Marinopyrrole A (maritoclax) is a selective Mcl-1 antagonist. This molecule is known to bind to Mcl-1 but not to Bcl-XL and to target Mcl-1 for proteasomal degradation.

VU661013 is a potent and selective Mcl-1 inhibitor with a Ki of 97±30 pM for human Mcl-1 in a TR-FRET assay. However, VU661013 does not significantly inhibit BCL-xL or BCL-2. VU661013 is a molecule known to destabilize the BIM/Mcl-1 association and induce apoptosis in AML.

S63845 is a potent and selective Mcl-1 inhibitor but is known not to bind to other BCL-2 components, BCL-2 or BCL-XL.

A-1210477 is a potent and selective Mcl-1 inhibitor and is known to have 100-fold or more selectivity than other Bcl-2 family members.

UMI-77 is a selective Mcl-1 inhibitor and is known to exhibit higher selectivity than other Bcl-2 family members.

Motixafortide (BL-8040, BKT140, TF 14016, 4-fluorobenzoyl, 4F-benzoyl-TN14003, T140) is a CXCR4 antagonist and is known to induce apoptosis in AML blasts by downregulating ERK, BCL-2, Mcl-1, and cyclin D1 via alteration of miR-15a/16-1 expression.

(R)-(-)-gossypol (AT-101) acetate, the R-(-) enantiomer of gossypol acetate, is a molecule that is known to bind to Bcl-2, Bcl-xL, and Mcl-1, and simultaneously induce apoptosis and cytoprotective autophagy.

Sabutoclax (BI-97C1) is a molecule known to be a pan-Bcl-2 inhibitor, including Bcl-xL, Bcl-2, Mcl-1, and Bfl-1.

TW-37 is a molecule known to be a non-peptide inhibitor against Bcl-2, Bcl-xL, and Mcl-1.

Gamboformic acid (guttatic acid, gutinic acid, beta-gutiferin) is a molecule known to activate caspases and competitively inhibit Bcl-XL, Bcl-2, Bcl-W, Bcl-B, Bfl-1, and Mcl-1.

Examples of the siRNA against the MCL1 gene that can be used in the present invention include, for example, an siRNA targeting the signal sequence of the MCL1 gene, an siRNA targeting the open reading frame sequence of the MCL1 gene, etc.. For example, a commercially available siRNA reagent (Dharmacon ON-TARGETplus siRNA SMARTPool) comprising a mixture of four siRNAs targeting different sites of the MCL1 gene can be used.

The present invention also found that some osteosarcoma cells demonstrate only slight amplification of the MCL1 gene, and instead demonstrate greatly increased amplification of the BCL2L1 gene or greatly increased production of Bcl-xL protein. It was revealed that the application of an Mcl-1 inhibitor to such osteosarcoma cells does not reduce cell viability, while the application of a Bcl-xL inhibitor reduces cell viability. Based on this finding, the present invention provides the second embodiment described above.

Examples of the Bcl-xL inhibitors that can be used in the pharmaceutical composition of the second embodiment of the present invention include, but are not limited to, A-1331852, siRNA against the BCL2L1 gene, etc.

A-1331852 is a potent and selective BCL-XL inhibitor, and is a compound that is believed to be potentially useful in the treatment of cancer, immune diseases, and autoimmune diseases.

Examples of the siRNA against the BCL2L1 gene that can be used in the present invention include, for example, an siRNA targeting the signal sequence of the BCL2L1 gene, an siRNA targeting the open reading frame sequence of the BCL2L1 gene, etc.. For example, a commercially available siRNA reagent (Dharmacon ON-TARGETplus siRNA SMARTPool) comprising a mixture of four types of siRNA targeting different sites of the BCL2L1 gene can be used.

In a third embodiment of the present invention, obatoclax, tipifarnib, bortezomib, carfilzomib, SN-38, and A-1331852 have different activities and their mechanisms of action are only partially clarified, but they all commonly have the ability to reduce the cell viability of osteosarcoma cells.

Tipifarnib is an extremely selective inhibitor of farnesyltransferase, an enzyme required for the activation of the proto-oncogene HRAS, and research for the clinical application of Tipifarnib is currently underway for head and neck squamous cell carcinoma etc..

Bortezomib is a proteasome inhibitor in myeloma cells and mantle cell lymphoma cells, and is commercially available as a drug for multiple myeloma, primary macroglobulinemia, and lymphoplasmocytic lymphoma. Carfilzomib is one of the selective proteasome inhibitors, and is commercially available as a drug for multiple myeloma. These proteasome inhibitors are compounds with a mechanism of action that induces cell cycle arrest and apoptosis of tumor cells by accumulating polyubiquitinated proteins, which are substrates for the proteasome, thereby suppressing tumor growth. However, the proteasome inhibitors show high toxicity to other types of cancer cell lines, such as colorectal and lung cancer, and do not have selectivity for osteosarcoma.

SN-38 is known as a topoisomerase inhibitor and to have antitumor effects; and as an active metabolite of irinotecan, and to have 1000 times more active than irinotecan itself.

In the pharmaceutical composition of the first embodiment of the present invention, any one of the Mcl-1 inhibitors comprised as active ingredients may be the active ingredient, or two or more Mcl-1 inhibitor compounds may be combined to form the active ingredient.

In the pharmaceutical composition of the second embodiment of the present invention, any one of the Mcl-1 inhibitors comprised as active ingredients may be the active ingredient, or two or more Bcl-xL inhibitor compounds may be combined to form the active ingredient.

In the pharmaceutical composition of the third embodiment of the present invention, any one of these six compounds comprised as active ingredients may be the active ingredient, or two or more compounds selected from the six compounds may be combined to form the active ingredient.

The pharmaceutical composition of the first embodiment, the pharmaceutical composition of the second embodiment, and the pharmaceutical composition of the third embodiment of the present invention (hereinafter simply referred to as the "pharmaceutical composition of the present invention") may be administered via any administration route, among which oral administration and intravenous administration are commonly used.

As shown in the examples of this application, the difference in drug sensitivity between obatoclax and navitoclax indicates that Mcl-1 is important for survival of the osteosarcoma cells and that simultaneous inhibition of Bcl-xL by Mcl-1 leads to efficient induction of apoptosis in osteosarcoma cells. In this study, MCL1 expression was upregulated in 10 of 11 osteosarcoma cell lines, and the copy number of MCL1 gene was increased in 5 of the cell lines.

It was known in the art that mutations in p53 pathway proteins have been identified in more than 75% of osteosarcomas, and that the PI3K/AKT/mTOR pathway promotes MCL1 expression at multiple levels, including transcription through activation of CREB transcription factor, activation of mTORC1 and subsequent phosphorylation of 4E-BP1, and proteasomal degradation through inhibition of GSK3-induced phosphorylation. These characteristic genomic structural abnormalities and genetic mutations indicate widespread enhancement of Mcl-1-dependent escape from programmed cell death in osteosarcoma. On the other hand, as shown in the examples of this application, G-292 cells with BCL2L1 amplification were highly sensitive to obatoclax as well as Bcl-xL-selective BH3 mimetics, suggesting that Mcl-1 and Bcl-xL complement each other in their functions in osteosarcoma.

Based on these findings, it has become clear that osteosarcoma to be treated by the pharmaceutical composition of the present invention is preferably one in which a copy number of the MCL1 gene is amplified, one in which a production of Mcl-1 protein is increased, or one in which a copy number of the BCL2L1 gene is amplified or a production of Bcl-xL protein is increased in the cells. Osteosarcoma having such properties is one in which cell proliferation is strongly inhibited by the five compounds that are the ingredients of the present invention, and, therefore, one in which a high therapeutic effect is obtained by administration of the pharmaceutical composition of the present invention.

The copy number of the MCL1 gene or the BCL2L1 gene in the cells can be measured using a general method for detecting nucleic acids in the art, for example, by PCR or FISH. When measured by PCR, the copy number can be measured by amplifying the gene using a part of the nucleotide sequence of the MCL1 gene or the BCL2L1 gene as a primer. When measured by FISH, the copy number can be measured by using a part of the nucleotide sequence of the MCL1 gene or the BCL2L1 gene as a probe.

The production of Mcl-1 protein or Bcl-xL protein in cells can be measured using a general method for detecting proteins in the art, for example, by immunoblotting or immunohistochemical staining. Specifically, when measured by immunoblotting, a sample of cell lysate is electrophoresed on an SDS-polyacrylamide gel, which is transferred to a PVDF membrane, and then proteins on the SDS-polyacrylamide gel were reacted with an antibody against Mcl-1 protein as a primary antibody, which is detected to measure.

### Pharmaceutical composition for treating osteosarcoma with a combination drug

In the examples of the present invention, other drugs that are effective in combination with Mcl-1 inhibitors such as obatoclax were screened, and Bcl-xL inhibitors or insulin-like growth factor 1 receptor (IGF-1R) inhibitors were identified as compounds that exhibit synergistic effects with Mcl-1 inhibitors such as obatoclax, AZD5991, and MIK665.

In the study of the examples of the present application, it was shown that the production of Mcl-1 protein and the production of Bcl-xL protein were enhanced in almost all osteosarcoma cell lines examined, suggesting that inhibition of the function of both molecules may be an effective combination therapy for the osteosarcoma.

In addition, it was known that IGF-1R activates the survival signal PI3K/Akt/mTOR pathway that confers resistance to apoptosis, and the chromosome 15q26.3 region on which IGF-1R is located is often amplified in the osteosarcoma. In the examples of the present invention, IGF treatment significantly increased the phosphorylation level of Akt, which is downstream of IGF-1R, resulting in abolishing obatoclax-induced apoptosis. Therefore, it is indicated that activation of the IGF-1R/PI3K/Akt/mTOR pathway plays an important role in the survival of osteosarcoma cells. Furthermore, in the examples of the present invention, it was found that either IGF1R or insulin receptor (IR) was expressed in osteosarcoma cells, suggesting that combination therapy of obatoclax with a dual inhibitor of IGF-1R and IR, such as OSI906, may be an effective combination therapy for the osteosarcoma.

Before the filing date of this application, an antitumor effect of an IGF-1R inhibitor alone against osteosarcoma was not known. However, in the examples of this application, it was shown that by using an active ingredient of the pharmaceutical composition in the first embodiment of the present invention, such as obatoclax, in combination with an IGF-1R inhibitor, the IGF-1R inhibitor has the enhancing effect on the action of the active ingredient of the pharmaceutical composition in the first embodiment of the present invention, such as obatoclax, against osteosarcoma.

In the examples of the present invention, based on these possibilities, it was found that, when an osteosarcoma cell line was treated with an Mcl-1 inhibitor in a combination with a Bcl-xL inhibitor or an IGF-1R inhibitor, the cell viability was significantly reduced.

Based on this finding, in the fourth embodiment, the present invention can also provide a pharmaceutical composition for treating osteosarcoma, which comprises an Mcl-1 inhibitor in combination with a Bcl-xL inhibitor or an IGF-1R inhibitor.

The examples of the Mcl-1 inhibitors and Bcl-xL inhibitors that can be used in the pharmaceutical composition of the present invention are as described above.

The examples of the IGF-1R inhibitors that can be used in the pharmaceutical composition of the present invention include, but are not limited to, OSI906, AEW541, AZD3463, and siRNA against the IGF-1R gene.

OSI906 is a potent, selective dual inhibitor of IGF-1R and insulin receptor (IR) kinases, that can be administered orally. It is known to inhibit IGF-1R autophosphorylation and to inhibit an activation of pathway downstream of Akt, ERK1/2, and S6 kinases, resulting in inhibiting the proliferation of tumor cell lines.

AEW541 is an inhibitor of IGF-1R and insulin receptor (IR) , that can be administered orally and is known to have antitumor activity.

AZD3463 is an inhibitor of ALK/IGF1R, that can be administered orally and that has suppressive effect on cell viability by inducing both cell apoptosis and autophagy.

As the siRNA against the IGF-1R gene, for example, siRNA targeting the signal sequence of the IGF-1R gene, siRNA targeting the open reading frame sequence of the IGF-1R gene, etc. can be used. For example, a commercially available siRNA reagent (Dharmacon ON-TARGETplus siRNA SMARTPool) comprising a mixture of four types of siRNA targeting different sites of the IGF-1R gene can be used.

In the pharmaceutical composition of the fourth embodiment of the present invention which is combined with an IGF-1R inhibitor, the osteosarcoma to be treated has the same characteristics as the osteosarcoma to be treated in the pharmaceutical composition of the first embodiment and the pharmaceutical composition of the second embodiment.

### Study of osteosarcoma treatment markers

As described above, the osteosarcoma to be treated in the present invention is characterized as a cell in which a copy number of the MCL1 gene is amplified or in which a production of Mcl-1 protein is increased, a cell in which a copy number of the BCL2L1 gene is amplified, or a production of Bcl-xL protein is increased. By examining whether osteosarcoma tissue or blood collected from a patient suffering from osteosarcoma has these characteristics, it can be confirmed whether the osteosarcoma in the patient is a target for treatment with the pharmaceutical composition of the present invention. In other words, these characteristics are indicators of whether the osteosarcoma in the patient is a target for administration of the pharmaceutical composition of the present invention.

Therefore, in a fifth embodiment, the present invention provides a method for detecting an increase in the copy number of the MCL1 gene or an increase in the production of the Mcl-1 protein, or an increase in the copy number of the BCL2L1 gene or an increase in the production of the Bcl-xL protein in osteosarcoma tissue or blood collected from a patient suffering from osteosarcoma, which is an indicator of whether or not the patient is a target for administration of a pharmaceutical composition comprising a component selected from the group consisting of an Mcl-1 inhibitor, obatoclax, tipifarnib, bortezomib, carfilzomib, SN-38, A-1331852, and derivatives thereof, or a combination thereof, or comprising an Mcl-1 inhibitor in combination with a Bcl-xL inhibitor or an IGF-1R inhibitor.

If this characteristic is present in osteosarcoma tissue or blood collected from a patient suffering from osteosarcoma, the pharmaceutical composition of the present invention is administered to the patient, but if this characteristic is not present, the effect of the pharmaceutical composition of the present invention may not be sufficient, and therefore a decision can be made to select a different treatment.

In this embodiment, the copy number of the MCL1 gene or the copy number of the BCL2L1 gene in the cells can be measured by PCR or FISH. In addition, the production of Mcl-1 protein or Bcl-xL protein in the cells can be measured by immunoblotting or immunohistochemical staining.

The present invention will be specifically described below with reference to examples. The examples shown below are not intended to limit the present invention in any way.

### Examples

### Example 1: Identification of molecular targeted drugs that inhibit the proliferation of osteosarcoma cells

In this example, compounds capable of inhibiting the proliferation of osteosarcoma cells were identified from among known molecular targeted drugs.

In order to identify molecular targeted drugs that broadly inhibit the proliferation of osteosarcoma cells, a library of inhibitors comprising 92 molecular targeted drugs was used to evaluate their effects on the proliferation of osteosarcoma cells, such as nine osteosarcoma cell lines available from public cell banks and two osteosarcoma patient-derived cell lines (PDC) established in the inventor's laboratory, and to perform drug screening.

The nine human osteosarcoma cell lines (MG-63 cells, NY cells, HOS cells, HuO9 cells, HuO-3N1 cells, SJSA-1 cells, NOS-1 cells, NOS-10 cells, and G-292 clone A141B1 (G-292) cells) were purchased from the American Type Culture Collection, the RIKEN BioResource Research Center, or the Japanese Collection of Research Bioresources Cell Bank (JCRC) and used at lower passage numbers.

Specifically,
- MG-63 cells, NY cells, and HOS cells were cultured in 96-well plates using MEM (Wako) comprising 10% FBS (CORNING), 1% non-essential amino acid solution (Wako), and 100 µ(micro)g/mL kanamycin (MEIJI),
- HuO9 cells, HuO-3N1 cells, SJSA-1 cells, NOS-1 cells, and NOS-10 cells were cultured in 96-well plates using RPMI-1640 medium (Wako) comprising 10% FBS and 100 µ(micro)g/mL kanamycin.
- G-292 clone A141B1 (G-292) cells were cultured in 96-well plate using McCoy's 5a medium comprising 10% FBS,
- Sa-xeno-147 and Sa-xeno-184 cell lines were developed from patient-derived xenograft tumors and were cultured in collagen-coated dishes with RPMI 1640/Ham's F12 (1:1) medium comprising 15% FBS, 20 mM HEPES, and antibiotic-antimycotic solution (Sigma-Aldrich). Cells were cultured at 37°C and 5% CO2.

Cells were seeded in triplicate in 96-well plates (Greiner) or type I collagen-coated plates (IWAKI) at a density of 2000 cells/well and cultured for at least 24 h. After 72 hours of incubation with any of the 96 compounds of the library of the inhibitors, cells were incubated with CellTiter-Glo reagent (Promega) for 2 minutes on a Titramax 100 plate shaker (Heidolph), and after 10 minutes of equilibration, cell viability assays of each osteosarcoma cell were performed by measuring luminescence using a Tristar LB941 multimode microplate reader (Berthold Technologies). The cell viability was calculated as a ratio of the DMSO control to evaluate their effects on osteosarcoma cell proliferation (n=2).

Data of the cell viability were analyzed using GraphPad Prism, and the average values of two independent experiments were graphically displayed as heatmaps using heatmap.2 with gplots package in ComplexHeatmap version 2.4.3 (R version 4.0.2).

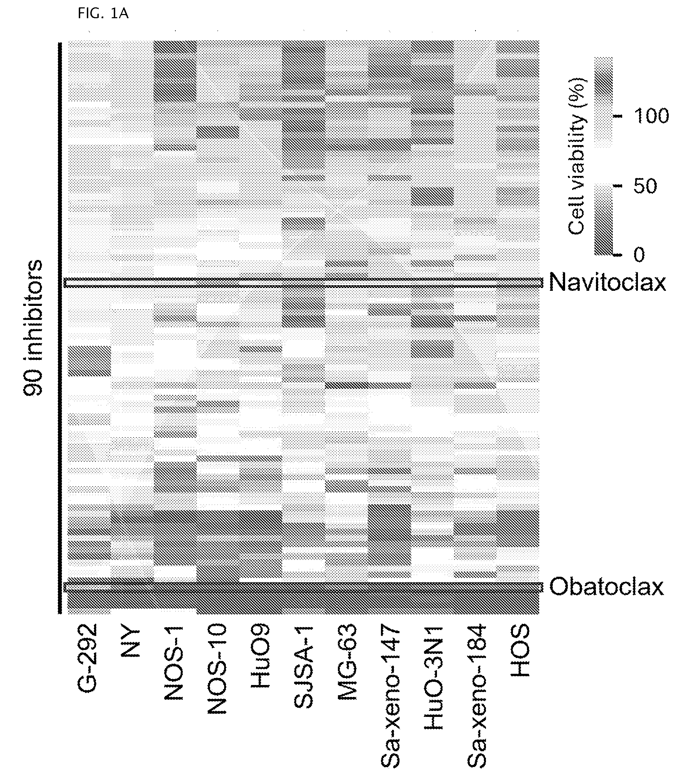

The effect of compounds on osteosarcoma cell proliferation is shown in Fig. 1A. Heatmap comparison of cell viability confirmed that five drugs (obatoclax, tipifarnib, bortezomib, carfilzomib, and SN-38) showed extensive inhibition of osteosarcoma cell proliferation.

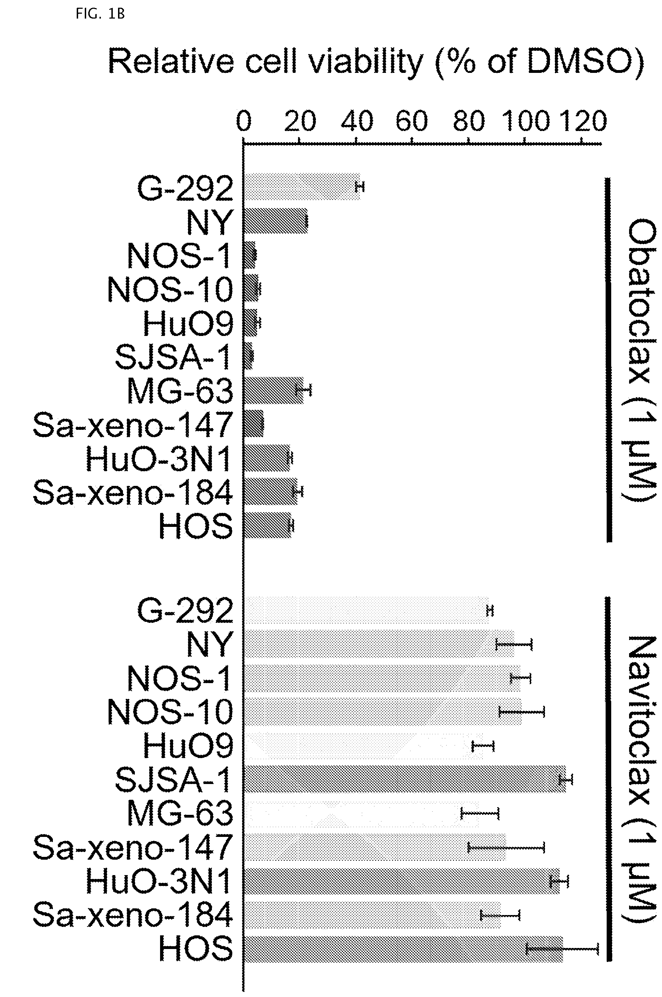

Among these five compounds, obatoclax, a pan-BH3 mimetic, was further investigated. Specifically, the osteosarcoma cells (the above described 11 types of osteosarcoma cells) were seeded in triplicate at a density of 2000 cells/well on 96-well plates (Greiner) or type I collagen-coated plates (IWAKI) and cultured for at least 24 hours. The osteosarcoma cells (i.e., the above described 11 types) were then treated with 1 µ(micro)M obatoclax for 72 hours, after which cell viability was measured using the CellTiter-Glo assay reagent. The results showed that obatoclax demonstrated a strong growth inhibitory effect on all osteosarcoma cell lines. In contrast, treatment with 1 µ(micro)M navitoclax, a dual BH3 mimetic, demonstrated little effect on osteosarcoma cell proliferation (Fig. 1B), indicating a significant difference in the drug sensitivity of osteosarcoma cells to BH3 mimetics.

Next, the expression of apoptosis-related proteins in obatoclax-treated osteosarcoma cells was confirmed by immunoblotting.

Specifically, cells were harvested using a cell scraper, lysed in SDS lysis buffer [100 mM Tris-HCl (pH 7.5), 10% glycerol, 1% SDS], and boiled for 10 min; protein concentration was measured using the Pierce^{™} BCA Protein Assay Kit (Thermo Fisher) after centrifugation at 20,000 x g for 10 min; equal amounts of cell lysates were electrophoresed on SDS-polyacrylamide gels; proteins were transferred to polyvinylidene difluoride (PVDF) membranes and immunoblotted with the following antibodies (primary antibodies):
Antibody against Mcl-1 (CST #5453);
Antibody against Bcl-xL (CST #2764);
Antibody against Bcl-2 (CST #4223);
Antibody against Bid (CST #2002);
Antibody against Bim (CST #2933);
Antibody against NOXA (CST #14766);
Antibody against Bax (CST #5023);
Antibody against Bak (CST #12105);
mAb against β-actin (Sigma #A1978).

Then, each antibody was detected using HRP-conjugated anti-mouse IgG or anti-rabbit IgG and enhanced chemiluminescence reagent (ECL prime, GE Healthcare). Chemiluminescence signals were detected by an AI600 image analyzer (GE Healthcare). In some experiments, cells were resuspended in NP-40 lysis buffer [20 mM Tris, 150 mM NaCl, 1% Nonidet P-40, 10% glycerol, 1 mM EDTA, 1 mM EGTA, and protease and phosphatase inhibitors], incubated on ice for 10 min, and centrifuged at 20,000 × g for 5 min.

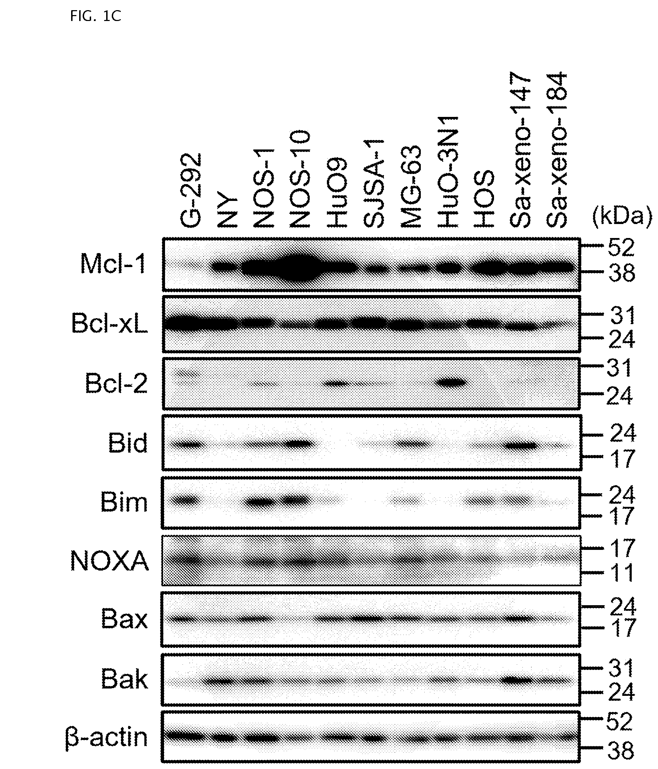

The results are shown in Fig. 1C. Obatoclax targets three anti-apoptotic Bcl-2 family proteins (i.e., Bcl-2, Bcl-xL, and Mcl-1), whereas navitoclax is more selective for Bcl-2 and Bcl-xL, suggesting that Mcl-1 is important for osteosarcoma cell survival. Immunoblotting showed that Mcl-1 was highly expressed in all osteosarcoma cell lines except G-292 cells and Bcl-xL was commonly expressed. Bcl-2 expression levels were below the detection limit in many osteosarcoma cells.

In osteosarcoma, it has been reported that the chromosome 1q21.2-3 region where MCL1 is located is often amplified, and an increase in the genomic copy number of MCL1 causes cancer progression. Therefore, the present inventors investigated the changes in the copy number of various IGF1R/PI3K/Akt/mTOR pathway-related genes, such as MCL1, TARS2, ENSA, PIP5K1A, and OTUD7B, located on the chromosome 1q21.2-3 region in each cell line. In addition, since the above results confirmed that Bcl-xL is also widely and highly expressed in osteosarcoma cell lines, in addition to Mcl-1, the present inventors investigated the changes in the copy number of MCL1 and BCL2L1.

Genomic DNA from each cell was prepared using ReverTra Ace qPCR RT Master Mix (TOYOBO) and DNeasy Blood & Tissue Kits (QIAGEN) according to the manufacturer's protocol for each cell. Quantitative real-time polymerase chain reaction (qPCR) was performed using FastStart Essential DNA Green Master (Roche) and Light Cycle 96 (Roche) by the ΔΔCt method. The copy number of each gene was quantified relative to the level of the repeat sequence element, LINE1 element, and DNA copy numbers were calculated and expressed as log₂ ratios. As a control, the changes in the copy number of human IGF1R located on chromosome 15q26.3 were examined. Data are shown as mean ± SD (n = 3).

The primer pairs for detecting the copy number of DNA were as follows:
Human MCL1, forward, 5'-cttccaaggtaagggggttc-3' (SEQ ID NO: 1);
Human MCL1, reverse, 5'-actgactcgtttccggtttcc-3' (SEQ ID NO: 2);
Human TARS2, forward, 5'-atgggacgtgtgttacctgc-3' (SEQ ID NO: 3);
Human TARS2, reverse, 5'-tagtccggggttccttctgt-3' (SEQ ID NO: 4);
Human ENSA, forward, 5'-ttcttctgtgttcgtccgca-3' (SEQ ID NO: 5);
Human ENSA, reverse, 5'-gacaccaacaggaaagggct-3' (SEQ ID NO: 6);
Human PIP5K1A, forward, 5'-agggaggatgggtggtaact-3' (SEQ ID NO: 7);
Human PIP5K1A, reverse, 5'-cgaatgttcttgccacctgc-3' (SEQ ID NO: 8);
Human OTUD7B, forward, 5'-acccctattgccctttggtg-3' (SEQ ID NO: 9);
Human OTUD7B, reverse, 5'-ctgccatgcaagaaagcgtt-3' (SEQ ID NO: 10);
Human BCL2L1, forward, 5'-cctctcccgacctgtgatac-3' (SEQ ID NO: 11);
Human BCL2L1, reverse, 5'-cttcctcggaaagtcactcc-3' (SEQ ID NO: 12);
LINE1 element, forward, 5'-aaagccgctcaactacatgg-3' (SEQ ID NO: 13);
LINE1 element, reverse, 5'-tgctttgaatgcgtcccagag-3' (SEQ ID NO: 14);
Human IGF1R, forward, 5'-atccgccattctcatgcctt-3' (SEQ ID NO: 15);
Human IGF1R, reverse, 5'-tttaacaaggcagggagggc-3' (SEQ ID NO: 16).

Human genomic DNA (PROMEGA) was used as a template for the PCR positive control.

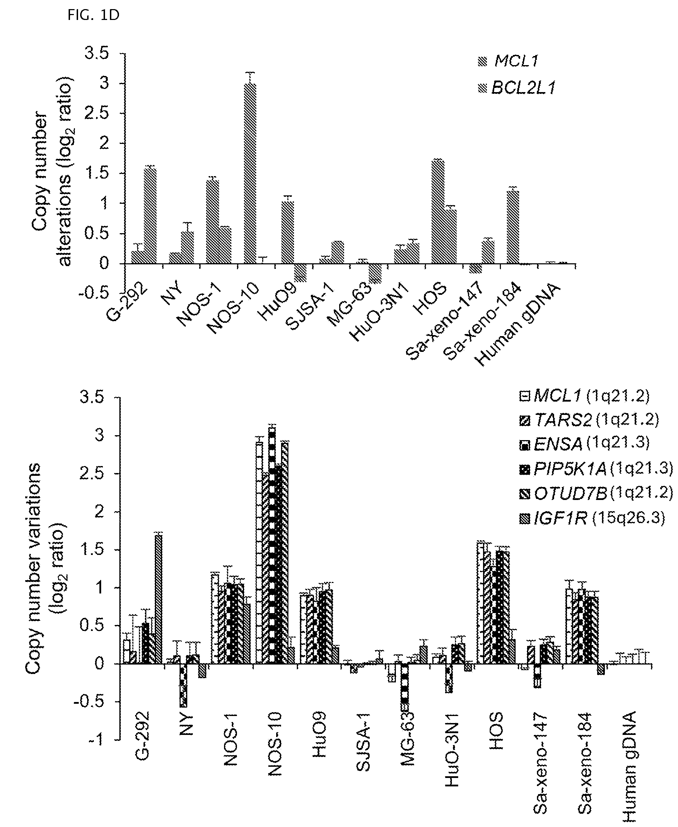

The results are shown in the upper and lower panels of Fig. 1D. The increase in the copy number of MCL1 was observed in osteosarcoma cell lines (5 of 11 osteosarcoma cell lines) at a high frequency of 45% using genomic DNA targeting qPCR analysis, and the increase in the copy number of BCL2L1 was observed in one of 11 osteosarcoma cell lines, G-292 cells (Fig. 1D).

Furthermore, FISH analysis for MCL-1 amplification was performed in NOS-10 cells and Sa-xeno-184 cells, in which MCL-1 amplification was observed, among the 5 cell lines in which the increase in the copy number of MCL-1 was shown. As a control, FISH analysis for MCL-1 amplification was similarly performed in Sa-xeno-147 cells and G-292 cells, in which no MCL-1 amplification was observed.

The status of the copy number of the MCL1 gene was examined on tissue microarrays or cell blocks of osteosarcoma cell lines and determined using in-house probes generated from BAC clones comprising ChromaTide TexasRed-12-dUTP (Thermo Fisher Science) (red) targeting the human MCL1 gene located on chromosome 1q21.2 and Fluorescein-12-dUTP (Roche) (green) targeting a sequence in the control chromosomal region 1p13.1. Images were acquired using an Olympus BX51 fluorescent microscope equipped with a charge-coupled device camera (DP71; Olympus, Tokyo, Japan). The increase in MCL1 gene was defined as the normal increase in MCL1 gene in the case of the MCL1/1p13.1 ratio ≥ 2.0, the high-level increase in the case of the MCL1/1p13.1 ratio ≥ 3.0, and the low-level increase in the case of 2.0 ≤ the MCL1/1p13.1 ratio was < 3.0.

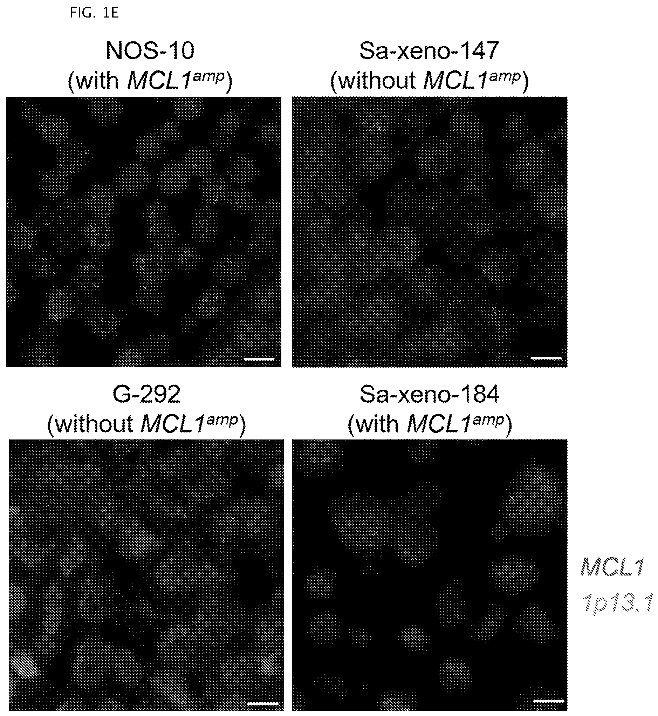

The results are shown in Fig. 1E. FISH analysis confirmed MCL1 amplification in NOS-10 cells and Sa-xeno-184 cells, but not in Sa-xeno-147 cells or G-292 cells (Fig. 1E). Scale bar: 20 µ(micro)m.

### Example 2: Discovery for other candidates of osteosarcoma therapeutic drug

In the present invention, since it was revealed that inhibition of Mcl-1 can suppress the viability of MCL1-amplified osteosarcoma cells, other compounds that can inhibit Mcl-1 were examined in this example.

In this example, the drug sensitivity of MIK665, an Mcl-1 inhibitor, was evaluated in NOS-10 cells. Various concentrations (i.e., 0, 1, 3, 10, 30, 100, 300, 1000, 3000, 10000, 30000 nM) of MIK665 (TargetMol) were administered, and cell viability was measured using CellTiter-Glo assay reagent 72 hours after drug treatment. As a control, osteosarcoma cells MG-63 cells, without amplification of the copy number of MCL1, were used. Data are shown as MG-63 mean ± SD (n = 3).

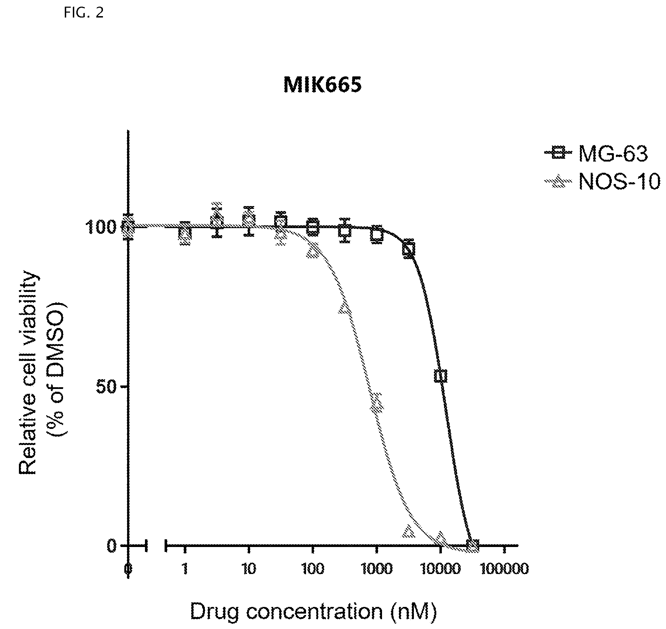

The results are shown in Fig. 2. It was found that treatment with low concentrations of MIK665 reduced cell viability in NOS-10 cells with MCL1 amplification, compared to MG-63 cells without MCL1 amplification.

### Example 3: Effect of Mcl-1 on survival of osteosarcoma cells

In this example, whether cell apoptosis is induced by inhibiting the expression of MCL1 was examined, in order to evaluate the effect of Mcl-1 on survival of osteosarcoma cells.

In this example, three types of cells were selected: NOS-10 cells in which MCL1 amplification was observed in Example 1, Sa-xeno-147 cells in which both MCL1 and BCL2L1 were observed to be normal in Example 1, and G-292 cells in which BCL2L1 amplification was observed in Example 1, and siRNA specific to BCL-2, BCL-xL, or MCL1 was introduced into these cells.

The following siRNAs were purchased from Dharmacon (Lafayette): SMARTpool siRNA for human BCL2 (L-003307-00-0005), SMARTpool siRNA for human BCL2L1 (L-003458-00-0005), and SMARTpool siRNA for human MCL1 (L-004501-00-0005). Silencer Negative Control No. 1 siRNA (Thermo) was used as a control.

Specifically, Opti-MEM I reduced serum medium (Thermo Fisher Scientific) comprising a final concentration of 2.4 nM of each siRNA and 1.0 µ(micro)L of Lipofectamine RNAiMAX transfection reagent (Thermo Fisher Scientific) was incubated at 24°C for 10 min. This was then mixed into cell suspension of each cell which was diluted to 2000 cells/100 µ(micro)L, and after 3 min of incubation at room temperature, each cell was seeded at 2000 cells/100 µ(micro)L/well in a collagen I-coated 96-well microplate with cap (IWAKI) for cell viability assays, or at 4×10⁵ cells/2 mL in a collagen I-coated 6-well microplate (IWAKI) for immunoblotting and qPCR. After 48 h of incubation, cell lysates were harvested.

The efficiency of RNAi-mediated gene knockdown of MCL1, BCL-xL, or BCL-2 was examined by immunoblotting in NOS-10 cell, Sa-xeno-147 cell, and G-292 cells (Fig. 3-1(A)-(C)) and by qPCR in Sa-xeno-147 cells and G-292 cells (Fig. 3-2) 48 hours after siRNA treatment. For immunoblotting, cells were immunoblotted with the following antibodies:
Antibody against Mcl-1 (CST #5453);
Antibody against Bcl-xL (CST #2764);
Antibody against Bcl-2 (CST #4223);
Antibody against PARP (CST #9542); and
Antibody against cleaved PARP (Asp214, CST #9541);
mAb against β-actin (Sigma #A1978).

The effect of the knockdown of Bcl-2 in Sa-xeno-147 cells and G-292 cells was examined at the mRNA level by RT-qPCR. The mRNA level was quantified relative to that of GAPDH. Data are shown as mean ± SD (n = 3). The primer pairs for detecting the expression of the BCL2 gene were as follows:
Human BCL2, forward, 5'-ccttctttgagttcggtggg-3' (SEQ ID NO: 17);
Human BCL2, reverse, 5'-tcttcagagacagccaggag-3' (SEQ ID NO: 18).
Human GAPDH, forward, 5'-atggggaaggtgaaggtcg-3' (SEQ ID NO: 19);
Human GAPDH, reverse, 5'-ggggtcattgatggcaacaata-3' (SEQ ID NO: 20).

The results are shown in Fig. 3-1 (A) to (C) and Fig. 3-2. In Fig. 3-2, data are shown as mean ± SD (n = 3). The results were determined by Student's t-test (**p < 0.01). In these figures, simultaneous inhibition of Mcl-1 and Bcl-xL induced apoptosis in the examined cells, indicating that the increase in the copy number of either MCL1 or BCL2L1 may be a potential vulnerability of osteosarcoma cells.

To further examine this possibility, the present inventors examined how caspase-3/7 activity changed in osteosarcoma cells (i.e., NOS-10 cells, Sa-xeno-147 cells, and G-292 cells) by knockdown of MCL1 with siRNA.

Caspase-3/7 activity was measured by the following method. Specifically, cells were incubated with Caspase-Glo 3/7 assay reagent (Promega) for 2 min on a Titramax 100 plate shaker (Heidolph) and, after 30 min of equilibration, were examined by measuring luminescence using a Tristar LB941 multimode microplate reader (Berthold Technologies). Relative cell viability was calculated as a ratio to the DMSO control.

The results are shown in Fig. 3-3. Data are shown as mean ± SD (n = 6). Dara were determined by Student's t-test (**p < 0.01). The levels of cleaved PARP and caspase-3/7 activity, apoptosis markers, were significantly increased in NOS-10 cells (Fig. 3-1(A) and Fig. 3-3(D)), but demonstrate almost no increase in Sa-xeno-147 cells (Fig. 3-1(B) and Fig. 3-3(E)) and G-292 cells (Fig. 3-1(C) and Fig. 3-3(F)). Furthermore, the levels of cleaved PARP and caspase-3/7 activity increased only in G-292 cells by BCL-xL knockdown. These results suggest that the increase in the copy number of either MCL1 or BCL2L1 may be a vulnerability of osteosarcoma cells.

These results indicate that, since simultaneous inhibition of Mcl-1 and Bcl-xL induces apoptosis, the present inventors examined the effect of double knockdown of BCL-xL and MCL1 on cell viability of osteosarcoma cells.

Specifically, three types of osteosarcoma cells (i.e., NOS-10 cells, Sa-xeno-147 cells, and G-292 cells) were treated with siRNAs for BCL-xL and MCL1, and cell viability was measured 48 hours after treatment using CellTiter-Glo Assay reagent.

Data are shown in Fig. 2-3. Data for siRNA treatment are shown as mean ± SD (n = 6). Data were determined by Student's t-test (**p < 0.01). The viability of all three types of osteosarcoma cells was significantly decreased (Fig. 3-4 (G) to Fig. 3-4 (I)). These results indicate that Bcl-xL and Mcl-1 play important roles in osteosarcoma cell survival, and that the increase in the copy number of either gene may be a potential molecular target in osteosarcoma cells.

The above results indicate that, since simultaneous inhibition of Mcl-1 and Bcl-xL induces apoptosis, the present inventors evaluated the effect of combined use of an Mcl-1 inhibitor and a Bcl-xL inhibitor on osteosarcoma cell survival.

In this example, AZD5991 or MIK665 was used as an Mcl-1 inhibitor, and A-1331852 was used as a Bcl-xL inhibitor. The results for AZD5991 are shown in Fig. 3-5, and the results for MIK665 are shown in Fig. 3-6.

These results show that A-1331852 alone had almost no effect on reduction of cell viability, but when used in combination with AZD5991 or MIK665, it had a strong effect on reduction of cell viability. In other words, it was shown that the combined use of an Mcl-1 inhibitor and a Bcl-xL inhibitor can be effective in treating osteosarcoma.

### Example 4: Suppression of viability of osteosarcoma cell by inhibition of Mcl-1 and Bcl-xL

In this example, the effect of anti-apoptotic Bcl-2 family proteins on pharmacological inhibition against osteosarcoma cell proliferation was examined.

In addition to obatoclax and navitoclax, the BH3 mimetics with high selectivity for Bcl-2, Bcl-xL, or Mcl-1 have been developed such as venetoclax, A-1331852, AZD5991, or MIK665, respectively. Osteosarcoma cells were treated with these BH3 mimetics to determine whether changes in cell viability occurred.

Specifically, four types of osteosarcoma cells (i.e., NOS-10 cells, Sa-xeno-147 cells, G-292 cells, and Sa-xeno-184 cells) were treated with six types of BH3 mimetics (i.e., obatoclax, navitoclax, venetoclax, A-1331852, AZD5991, and MIK665), and the cell viability of the osteosarcoma cells was measured.

The results are shown in Fig. 4-1. In NOS-10 cells and Sa-xeno-184 cells with MCL1 amplification, AZD5991 and MIK665 were found to reduce cell viability at relatively low concentrations, similar to obatoclax treatment (Fig. 4-1 (A)). A-1331852 was most effective in reducing cell viability in G-292 cells with BCL2L1 amplification, whereas AZD5991 or MIK665 showed moderate inhibitory effects in Sa-xeno-147 cells (Fig. 4-1(B) and Fig. 4-1(C)).

Meanwhile, the combined effect of AZD5991 and 3 µ(micro)M Navitoclax on cell viability of NOS-10 cells was examined. Cell viability was measured using CellTiter-Glo assay reagent 72 hours after drug treatment. Data are shown as mean ± SD (n = 3). The combination of AZD5991 and Navitoclax increased the inhibitory effect on cell viability in NOS-10 cells (Fig. 4-2 (D)). This indicates that simultaneous inhibition of Bcl-xL and Mcl-1 is expected to induce higher growth inhibition than inhibition of Mcl-1 alone in MCL1-amplified osteosarcoma cells.

Furthermore, since it has been found that the increase in the copy number of MCL1 frequently occurs in lung adenocarcinoma and can be a therapeutic target, the inventors of the present invention decided to compare the IC50 value of obatoclax in lung adenocarcinoma cell lines and that in osteosarcoma cell lines.

In this example, G-292 cells, NY cells, NOS-1 cells, NOS-10 cells, HuO9 cells, SJSA-1 cells, MG-63 cells, HuO-3N1 cells, HOS cells, Sa-xeno-147 cells, and Sa-xeno-184 cells were used as osteosarcoma cell lines, and LC-2/ad cells, H2228 cells, H226 cells, H3122 cells, and A549 cells were used as lung adenocarcinoma cell lines.

After 72 hours from treatment with obatoclax, the cell viability of each cell was measured using the CellTiter-Glo Assay reagent. The IC50 value was calculated using Prism software. The maximum concentration of obatoclax in serum (Cmax), 176 nM, is indicated by a dotted line.

The results are shown in Fig. 4-3. The mean IC50 value of obatoclax in osteosarcoma cell lines was lower than that in lung adenocarcinoma cell lines and was lower than the maximum serum concentration of obatoclax (Cmax), 176 nM. This means that the therapeutic effect of obatoclax against osteosarcoma can be achieved in vivo.

On the other hand, the cell viability after treatment with obatoclax in lung adenocarcinoma cell lines was highly variable and no specific trend could be observed. These results suggest that the presence of the increase in the copy number of anti-apoptotic Bcl-2 family proteins can be used to estimate the specific inhibitory effect of BH3 mimetics on osteosarcoma cell survival.

Various BH3 mimetics (e.g., navitoclax, obatoclax, venetoclax, A-1331852, and AZD5991) are currently being investigated as a combination therapy with standard therapy, and venetoclax in combination with cytarabine or hypomethylating agents demonstrates great clinical success in acute myeloid leukemia. Doxorubicin (DOX), cisplatin (CDDP), methotrexate (MTX), and ifosfamide (IFO) are used as standard therapy for osteosarcoma. IFO is a prodrug alkylating agent that is metabolized and activated in the body by hepatic cytochrome P450.

With reference to these findings, the present inventors investigated whether DOX, CDDP, or MTX, when combined with obatoclax, would demonstrate synergistic inhibition on proliferation of osteosarcoma cells in vitro. Specifically, the present inventors investigated the combined effects of 40 nM obatoclax with DOX (Fig. 4-4(E), Fig. 4-5(A)), with CDDP (Fig. 4-4(F), Fig. 4-5(B)), and with MTX (Fig. 4-4(G), Fig. 4-5(C)) on cell viability in NOS-10 cells and Sa-xeno-147 cells. Cell viability was measured for each cell 72 hours after each drug treatment using CellTiter-Glo assay reagent. All data are shown as mean ± SD (n = 3). IC50 values were calculated and the data were graphed using GraphPad Prism.

The results showed that the IC50 values of DOX, CDDP, or MTX were not decreased even in the presence of obatoclax (Fig. 4-4(E) to Fig. 4-4(G) and Fig. 4-5). This suggests that standard chemotherapy agents for osteosarcoma may not induce a synergistic cytotoxic effect when combined with obatoclax.

### Example 5: Discovery of suitable combination partners of obatoclax for osteosarcoma treatment

In this example, suitable combination partners of Mcl-1 inhibitors for osteosarcoma treatment were identified.

To identify suitable combination partners of Mcl-1 inhibitors such as obatoclax for osteosarcoma treatment, further drug screening was performed using an in-house library of inhibitors in the presence of obatoclax in osteosarcoma cells. Specifically, the effect of combination of 35 nM obatoclax with an inhibitor out of 94 molecular targeted drugs in the library on cell viability of NOS-10 cells was examined. Cell viability was measured using CellTiter-Glo assay reagent 72 hours after the cells were treated with each combination of drugs. All data are shown as mean ± SD (n = 3). GraphPad Prism was used to calculate IC50 values and graph the data.

As a result, it was revealed that IGF-1R inhibitors such as OSI906, AEW541, and AZD3463, when used in combination with Mcl-1 inhibitors such as obatoclax, AZD5991, and MIK665, enhanced the growth inhibitory effect and significantly reduced the IC50 value (Fig. 5-1).

Immunoblotting analysis was performed on various osteosarcoma cell lines using the following antibodies:
Antibody against IGF-1Rβ (CST #3027);
Antibody against insulin receptor β (CST #3025);
mAb against β-actin (Sigma #A1978).

It was demonstrated that at least one of IGF-1R and insulin receptor (IR) was expressed in the osteosarcoma cell lines (Fig. 4B).

Based on this finding, the present inventors next investigated the effects of combined treatment of obatoclax with OSI906, a dual inhibitor of IGF-1R and insulin receptors, on osteosarcoma cells in NOS-10 cells and Sa-xeno-147 cells.

That is, NOS-10 cells and Sa-xeno-147 cells were pretreated with 40 nM obatoclax or 4 µ(micro)M OSI906, and then treated with 40 ng/mL IGF. Cell samples were harvested 24 hours after drug treatment. The expression of each protein in these cells was examined by the following antibodies:
Antibody against IGF-1Rβ (CST #3027);
Antibody against p-Akt (S473, CST #4060);
Antibody against Akt (CST #4691);
Antibody against p-PRAS40 (CST #2997)
Antibody against PRAS40 (CST #2691)
Antibody against p-S6 (CST #5364)
Antibody against S6 (CST #2217)
Antibody against p-ERK (CST #9101)
Antibody against ERK (CST #9102)
Antibody against Mcl-1 (CST #5453);
Antibody against Bcl-xL (CST #2764);
Antibody against Bax (CST #5023);
Antibody against PARP (CST #9542); and
Antibody against cleaved PARP (Asp214, CST #9541);
mAb against β-actin (Sigma #A1978).

The results showed that the combination of obatoclax and OSI906 suppressed the phosphorylation levels of downstream molecules of IGF-1R, such as Akt, PRAS40, and S6 protein, and increased the amount of cleaved PARP. Moreover, the expression levels of phosphorylated-ERK, Mcl-1, Bcl-xL, and Bax did not change under these conditions (Fig. 5-3(B) and Fig. 5-3(C)). Moreover, the obatoclax-induced increase in the cleaved PARP was counteracted by IGF ligand stimulation, which was overcome in the presence of OSI906 (Fig. 5-2(C) and Fig. 5-3(C)).

It is suggested that the combination of OSI906 and obatoclax enhances obatoclax-induced apoptosis by overcoming drug resistance caused by activation of IGF signaling in osteosarcoma cells. To further examine this possibility, NOS-10 cells were pretreated with 40 nM obatoclax and/or 4 µ(micro)M OSI906 for 30 min, and then treated with 40 ng/mL IGF-1 to examine how the activity of caspase-3/7, an apoptotic signal, changes.

Caspase activity was measured using Caspase-Glo 3/7 assay reagent 24 hours after IGF-1 treatment. Data are shown as mean ± SD (n = 4). **p < 0.01 was determined by Student's t-test.

The results are shown in Fig. 5-4. The present inventors found that activation of IGF signaling which is associated with the addition of IGF-1 significantly inhibited obatoclax-induced apoptosis and conferred drug resistance in NOS-10 cells. This drug resistance could be overcome by the combined use of obatoclax and OSI906, indicating that the combined use of obatoclax and OSI906 enhanced obatoclax-induced apoptosis.

These results indicate that the combination of an IGF-1R inhibitor such as OSI906 with obatoclax enhanced obatoclax-induced apoptosis and synergistically reduced osteosarcoma cell viability by overcoming drug resistance mediated by IGF signaling activation in osteosarcoma cells.

### Example 6: In vivo study of combination of Mcl-1 inhibitor and OSI906

In this example, the present inventors investigated whether combination of an Mcl-1 inhibitor and OSI906 synergistically suppresses the proliferation of MCL1-amplified osteosarcoma tumors in a mouse xenograft model.

Using obatoclax or AZD5991 as an Mcl-1 inhibitor, the present inventors used mouse xenograft models of osteosarcoma with MCL1-amplified osteosarcoma (NOS-10 cells) and without MCL1-amplified osteosarcoma (Sa-xeno-147 cells) to determine the efficacy of the combination of obatoclax or AZD5991 with OSI906. To prevent side effects such as weight loss, the drugs were administered three times a week at relatively low concentrations (experimental schemes are shown in Figures 5A and 5D).

The changes in tumor volume of NOS-10 cells and Sa-xeno-147 cells by treatment of drugs was examined as follows. NOS-10 cells or Sa-xeno-147 cells were subcutaneously inoculated into immunodeficient SCID beige mice (CB17.Cg-PrkdcscidLystbg-J/CrlCrlj, Charles River Laboratories, Inc.) (5-6×10⁶ cells/mouse or 1×10⁶ cells/mouse) and orally treated three times a week with vehicle (30% PEG400, 0.5% Tween80, 5% propylene glycol in water), with obatoclax (5 mg/kg), with OSI906 (25 mg/kg), or with obatoclax + OSI906 (5 mg/kg and 25 mg/kg, respectively). The combination of AZD5991 and OSI906 were applied with vehicle (30% HP-β-CD in water, pH 9.0) corresponding to AZD5991, with vehicle (30% PEG400, 0.5% Tween80, 5% propylene glycol in water) corresponding to OSI906, with AZD5991 (30 mg/kg), with OSI906 (25 mg/kg), or with AZD5991 + OSI906 (30 mg/kg and 25 mg/kg, respectively). AZD5991 was administered intraperitoneally once a week, and OSI906 was administered orally five times a week. All data are shown as mean ± SD (n = 4, n = 5, or n = 6). *p < 0.05 and **p < 0.01 are determined using the Mann-Whitney U test.

The results using obatoclax as an Mcl-1 inhibitor are shown in Fig. 5B and Fig. 5C, and the results using AZD5991 are shown in Fig. 5D. In the NOS-10 cell xenograft model, obatoclax or OSI906 treatment alone had little effect on tumor growth. However, the combination of obatoclax and OSI906 significantly suppressed tumor growth (Fig. 6-2 (B)). However, the treatments did not reduce tumor growth of Sa-xeno-147 cells (Fig. 6-2 (C)). These results suggest that the combination of obatoclax and OSI906 may be a therapeutic intervention for MCL1-amplified osteosarcoma.

### Example 7: Genomic copy number and protein expression of MCL1 in patients with osteosarcoma

In this example, the genome copy number and protein expression of MCL1 in formalin-fixed paraffin-embedded specimens from patients with osteosarcoma were evaluated by FISH analysis and immunohistochemical staining analysis using tissue microarrays.

Formalin-fixed paraffin-embedded specimens from patients with osteosarcoma were used as samples, which were previously collected from 41 patients with osteosarcoma. Small tissue pieces were taken from the tissues of the formalin-fixed paraffin-embedded specimens using a 2 mm stainless steel biopsy needle (manual tissue puncher; Beecher Instruments, Silver Spring, Maryland). The tissue pieces were placed in a recipient array block with standard size. After the construction of the array block, multiple 4 µ(micro)m sections were prepared and subjected to fluorescence in situ hybridization (FISH) analysis and immunohistochemical staining analysis as described below.

To analyze the genome copy number of the human MCL1 gene and to measure the MCL1 copy number/1p13.1 ratio for samples derived from 41 osteosarcoma patients, FISH targeting the human MCL1 gene and sequences in the chromosomal region 1p13.1 were performed. The FISH was performed according to the method described in Example 1. As in Example 1, the increase in MCL1 gene was defined as an MCL1/1p13.1 ratio ≥ 2.0, the high-level increase was defined as MCL1/1p13.1 ratio ≥ 3.0, and the low-level icrease was defined as 2.0 ≤ MCL1/1p13.1 ratio < 3.0.

As a result, it was revealed that, of the 41 patients examined, 14.6% (n = 6) had the high-level MCL1 increase, and 31.7% (n = 13) had the low-level MCL1 increase (Fig. 7-1). The results of FISH are shown as examples for three samples (GTA479-19, GTA525-19, and GTA476-30) defined as having the high levels of MCL1 amplification (with MCL1amp) and one sample (GTA525-2) defined as having no amplification (without MCL1amp) (Fig. 7-2 (A)).

Furthermore, to confirm the expression of Mcl-1 protein, the expression of Mcl-1 protein was examined by immunohistochemical staining using the following method in the specimens in which MCL1 was shown to be amplified by FISH analysis. That is, antigen retrieval was performed by heating the slides of thin sections in an antigen retrieval solution (Nichirei Biosciences, Inc., Tokyo, Japan) at 95°C for 30 minutes. The sections were then reacted with a 1:200 diluted primary antibody, anti-Mcl-1 monoclonal antibody (CST, clone D5V5L), to form an antigen-antibody complex, which was visualized using Histofine Simple Stain MAX PO detection reagent (Nichirei Biosciences, Inc.). The staining procedure was performed using a Histostainer 36A automated staining system (Nichirei Biosciences, Inc.).

The results are shown in Fig. 7-3 (B). Scale bar: 20 µ(micro)m. As a result, Mcl-1 was detectable at the protein level in three samples (GTA479-19, GTA525-19, and GTA476-30) defined by FISH as having the high level increase in MCL1 amplification (with MCL1amp), whereas Mcl-1 was not detectable at the protein level in one sample (GTA525-2) defined by FISH as having no MCL1 amplification (without MCL1amp).

The results indicate that the amplification of the copy number of the MCL1 gene detected at the nucleic acid level in osteosarcoma patient samples is consistent with the detection of Mcl-1 protein at the protein level in osteosarcoma patient samples, and may be used as a biomarker to predict the efficacy of obatoclax treatment or the combination treatment of obatoclax and OSI906 in patients.

### Industrial Applicability

The present invention has demonstrated that compounds capable of suppressing osteosarcoma cell proliferation can be found, and, therefore, can provide a new pharmaceutical composition for treating osteosarcoma. In addition, it has been found that osteosarcoma, which is the target of treatment with the new pharmaceutical composition, is a cell in which a copy number of the MCL1 gene is amplified or in which a production of Mcl-1 protein is increased, or a cell in which a copy number of the BCL2L1 gene is amplified or in which a production of Bcl-xL protein is increased, and it has also been demonstrated that detection of these can be used as therapeutic markers for examining the therapeutic effect of the new pharmaceutical composition when treating osteosarcoma.

## Claims

1. A pharmaceutical composition for treating osteosarcoma, comprising an Mcl-1 inhibitor.

2. The pharmaceutical composition according to claim 1, wherein the Mcl-1 inhibitor is selected from the group consisting of obatoclax, AZD5991, MIK665, siRNA against the MCL1 gene, or a combination thereof.

3. The pharmaceutical composition according to claim 1 or 2, wherein the osteosarcoma is one in which a copy number of the MCL1 gene is amplified or one in which a production of the Mcl-1 protein is increased.

4. The pharmaceutical composition according to claim 3, wherein the copy number of the MCL1 gene in the cells is measured by PCR or FISH.

5. The pharmaceutical composition according to claim 3, wherein the production of Mcl-1 protein in the cells is measured by immunoblotting or immunohistochemical staining.

6. A pharmaceutical composition for treating osteosarcoma in which an Mcl-1 inhibitor is ineffective, comprising a Bcl-xL inhibitor.

7. The pharmaceutical composition according to claim 6, wherein the Bcl-xL inhibitor is selected from the group consisting of A-1331852, an siRNA against the BCL2L1 gene, or a combination thereof.

8. The pharmaceutical composition according to claim 6 or 7, wherein the Mcl-1 inhibitor is selected from the group consisting of obatoclax, AZD5991, MIK665, siRNA against the MCL1 gene, or a combination thereof.

9. The pharmaceutical composition according to claim 6 or 7, wherein the osteosarcoma is a cell in which a copy number of the BCL2L1 gene is amplified or one in which a production of the Bcl-xL protein is increased.

10. The pharmaceutical composition according to claim 9, wherein the copy number of the BCL2L1 gene in the cells is measured by PCR or FISH.

11. The pharmaceutical composition according to claim 9, wherein the production of Bcl-xL protein in the cells is measured by immunoblotting or immunohistochemical staining.

12. A pharmaceutical composition for treating osteosarcoma, comprising a component selected from the group consisting of obatoclax, tipifarnib, bortezomib, carfilzomib, SN-38, A-1331852, and derivatives thereof, or combinations thereof.

13. The pharmaceutical composition according to claim 12, wherein the osteosarcoma is a cell in which a copy number of the MCL1 gene is amplified or in which a production of the Mcl-1 protein is increased, or a cell in which a copy number of the BCL2L1 gene is amplified or in which a production of the Bcl-xL protein is increased.

14. The pharmaceutical composition according to claim 13, wherein the copy number of the MCL1 gene or the copy number of the BCL2L1 gene in the cells is measured by PCR or FISH.

15. The pharmaceutical composition of claim 13, wherein the production of Mcl-1 protein or Bcl-xL protein in the cells is measured by immunoblotting or immunohistochemical staining.

16. A pharmaceutical composition for treating osteosarcoma, comprising an Mcl-1 inhibitor in combination with a Bcl-xL inhibitor or an IGF-1R inhibitor.

17. The pharmaceutical composition of claim 16, wherein the Mcl-1 inhibitor is selected from the group consisting of obatoclax, AZD5991, MIK665, siRNA against the MCL1 gene, or a combination thereof.

18. The pharmaceutical composition according to claim 16, wherein the Bcl-xL inhibitor is selected from the group consisting of A-1331852 and siRNA against the BCL2L1 gene.

19. The pharmaceutical composition according to claim 16, wherein the IGF-1R inhibitor is selected from the group consisting of OSI906, AEW541, AZD3463 and siRNA against the IGF-1R gene.

20. The pharmaceutical composition according to any one of claims 16 to 19, wherein the osteosarcoma is a cell in which a copy number of the MCL1 gene is amplified or in which a production of the Mcl-1 protein is increased, or a cell in which a copy number of the BCL2L1 gene is amplified or in which a production of the Bcl-xL protein is increased.

21. The pharmaceutical composition according to claim 20, wherein the copy number of the MCL1 gene or the BCL2L1 gene in the cells is measured by PCR or FISH.

22. The pharmaceutical composition according to claim 20, wherein the production of the Mcl-1 protein or the Bcl-xL protein in the cells is measured by immunoblotting or immunohistochemical staining.

23. A method for detecting an increase in the copy number of the MCL1 gene or an increase in the production of the Mcl-1 protein, an increase in the copy number of the BCL2L1 gene, or an increase in the production of the Bcl-xL protein in osteosarcoma tissue or blood collected from a patient suffering from osteosarcoma, which is an indicator of whether or not the patient is a target for administration of a pharmaceutical composition comprising a component selected from the group consisting of an Mcl-1 inhibitor, a Bcl-xL inhibitor, obatoclax, tipifarnib, bortezomib, carfilzomib, SN-38, A-1331852, and derivatives thereof, or a combination thereof, or comprising an Mcl-1 inhibitor in combination with a Bcl-xL inhibitor or an IGF-1R inhibitor.

24. The method according to claim 23, wherein the copy number of the MCL1 gene or the BCL2L1 gene in the cells is measured by PCR or FISH.

25. The method according to claim 23, wherein the production of the Mcl-1 protein or the Bcl-xL protein in the cells is measured by immunoblotting or immunohistochemical staining.
